# EUROPÄISCHE PATENTSCHRIFT

(11) **EP 0 799 228 B1**
(45) Veröffentlichungstag und Bekanntmachung des Hinweises auf die Patenterteilung: **19.03.2003**
(21) Anmeldenummer: 95942100.9
(22) Anmeldetag: 11.12.1995
(51) Int. Cl.: C07D 493/10, C07D 495/10, C07D 491/10, C07D 309/10, C07D 335/02, C07D 333/38, C07D 211/66, A01N 43/16, A01N 43/08, A01N 43/18, A01N 43/10, A01N 43/40

(54) **3-ARYL-TETRONSÄURE-DERIVATE, DEREN HERSTELLUNG UND DEREN VERWENDUNG ALS SCHÄDLINGSBEKÄMPFUNGSMITTEL**
3-ARYL-TETRONIC ACID DERIVATIVES, THE PRODUCTION THEREOF AND THE USE THEREOF AS ANTI-PARASITIC AGENTS
DERIVES DE L'ACIDE 3-ARYL-TETRONIQUE, LEUR FABRICATION ET LEUR UTILISATION COMME PESTICIDES

(30) Priorität: 23.12.1994 DE 4446335; 02.11.1995 DE 19540736
(43) Veröffentlichungstag der Anmeldung: 08.10.1997
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); BECK, Gunther, D-51375 Leverkusen (DE); HAGEMANN, Hermann, D-51375 Leverkusen (DE); ERDELEN, Christoph, D-42799 Leichlingen (DE); WACHENDORFF-NEUMANN, Ulrike, D-56566 Neuwied (DE); ANDERSCH, Wolfram, D-51469 Bergisch Gladbach (DE); MENCKE, Norbert, D-51381 Leverkusen (DE); TURBERG, Andreas, D-40699 Erkrath (DE)
(86) Internationale Anmeldenummer: EP9504869
(87) Internationale Veröffentlichungsnummer: WO96020196

(56) Entgegenhaltungen:
- EP-A- 0 528 156
- EP-A- 0 647 637
- EP-A- 0 668 267
- WO-A-95/01971
- DE-A- 3 913 757
- DE-C- 92 589
- THE JOURNAL OF GENERAL CHEMISTRY OF THE U.S.S.R., Bd. 26, Nr. 10, Oktober 1956 Seiten 3891-3898, XP 000565019 NAZAROV I.N. ET AL. '47. Synthetic anesthetic substances. X. Esters of 1,2,5-trimethyl-4-carbalkoxy-4-piperidols. New synthetic analogs of a .alpha.-cocaine and .alpha.-eucaine'
- JOURNAL OF GENERAL CHEMISTRY OF THE USSR, Bd. 30, Nr. 12, Dezember 1960 Seiten 3883-3887, XP 000565020 UNKOVSKII B.V. ET AL. 'Esters of 1,3-dimethyl- and 1,2,3-trimethyl-4-carbomethoxy-4-piperidol s. New analogs of .alpha.-cocaine and .alpha.-eucaine'

## Beschreibung

Die vorliegende Erfindung betrifft neue 3-Aryl-4-hydroxy-Δ³-dihydro-furanon-Derivate (3-Aryl-tetronsäure-Derivate), Verfahren zu ihrer Herstellung und ihre Verwendung als Schädlingsbekämpfungsmittel.

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-528 156 bekannt, jedoch ist die dort beschriebene Wirkung nicht immer ausreichend.

EP 0 647 637 A beschreibt 3-Aryl-4-hydroxy-Δ³-dihydro-furanon-Derivate, Verfahren zu ihrer Herstellung und ihre Verwendung als Schädlingsbekämpfungsmittel.

DE 37 13 757 A beschreibt Zwischenprodukte mit 3-Aryl-4-alkoxy-Δ³-dihydrofuranon-Grundstruktur.

WO 95 01971 A beschreibt 3-Aryl-4-hydroxy-Δ³-dihydro-furanon-Derivate als Zwischenverbindungen zur Herstellung von 3-Aryl-4-alkoxy-Δ³-dihydro-furanon-Derivaten mit pestizider Wirkung.

EP 0 668 267 A beschreibt 1H-3-Aryl-pyrrolidin-2,4-dion-Derivate, Verfahren zu ihrer Herstellung und ihre Verwendung als Schädlingsbekämpfungsmittel.

Es wurden nun neue 3-Aryl-4-hydroxy-Δ³-dihydrofuranon-Derivate der Formel (I) 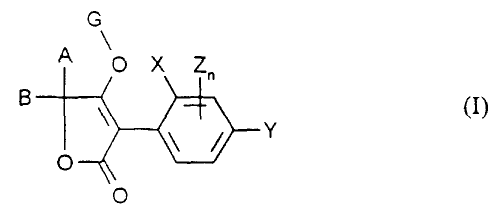 gefunden, in welcher
- A und B: für gegebenenfalls einfach oder mehrfach, gleich oder verschieden durch Halogen, C₁-C₈-Alkyl, C₃-C₈-Cycloalkyl, C₁-C₈-Halogenalkyl, C₁-C₆-Alkoxy, C₁-C₆-Alkylthio oder Phenyl substituiertes C₄-C₆-Alkandiyl stehen, in welchem ein oder zwei nicht direkt benachbarte Kohlenstoffatome durch die Gruppe 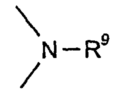 und/oder Sauerstoff und/oder Schwefel ersetzt sind,
- X: für C₁-C₆-Alkyl, Halogen oder C₁-C₆-Alkoxy steht,
- Y: für Wasserstoff, C₁-C₆-Alkyl, Halogen, C₁-C₆-Alkoxy oder C₁-C₃-Halogenalkyl steht,
- Z: für C₁-C₆-Alkyl, Halogen oder C₁-C₆-Alkoxy steht,
- G: für Wasserstoff (a) oder für eine der Gruppen 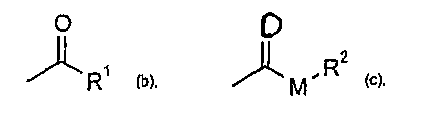 steht, in welchen
M für Sauerstoff oder Schwefel steht,
- R¹: für jeweils gegebenenfalls einfach oder mehrfach durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₂-C₈-alkyl oder gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls einfach bis fünffach durch Halogen, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls einfach bis fünffach durch Halogen, C₁-C₆-Alkyl, C ₁-C₆-Alkoxy, C₁-C₆-Halogenalkyl oder C₁-C₆-Halogenalkoxy substituiertes Phenyl-C₁-C₆-alkyl,
für jeweils gegebenenfalls einfach oder zweifach durch Halogen oder C₁-C₆-Alkyl substituiertes Pyridyl, Thienyl, Furanyl, Pyrazolyl, Pyrimidyl oder Thiazolyl,
für gegebenenfalls einfach bis dreifach durch Halogen oder C₁-C₆-Alkyl substituiertes Phenoxy-C₁-C₆-alkyl oder
für jeweils gegebenenfalls einfach oder zweifach durch Halogen, Amino oder C₁-C₆-Alkyl substituiertes Pyridinyloxy-C₁-C₆-alkyl, Pyrimidinyloxy-C₁-C₆-alkyl oder Thiazolyloxy-C₁-C₆-alkyl steht,
- R²: für jeweils gegebenenfalls einfach oder mehrfach durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls einfach oder mehrfach durch Halogen, C₁-C₄-Alkyl oder C₁-C₄-Alkoxy substituiertes C₃-C₈-Cycloalkyl oder
für jeweils gegebenenfalls einfach bis dreifach durch Halogen, Nitro, C₁-C₆-Alkyl, C₁-C₆-Alkoxy oder C₁-C₆-Halogenalkyl substituiertes Phenyl oder Benzyl steht,
- R⁹: für Wasserstoff, Q, COQ oder CO₂Q steht,
wobei
Q die oben für R² genannten Bedeutungen annehmen kann und
- n: 0, 1 oder 2 steht.

Unter Einbeziehung der verschiedenen Bedeutungen (a), (b) und (c), der Gruppe G ergeben sich folgende hauptsächlichen Strukturen (Ia) bis (Ic): 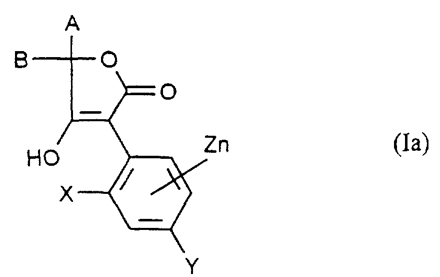 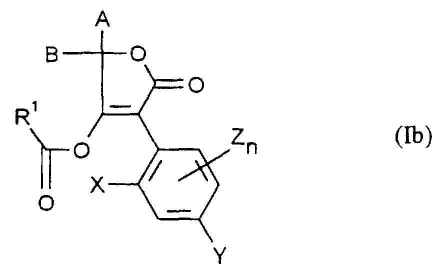 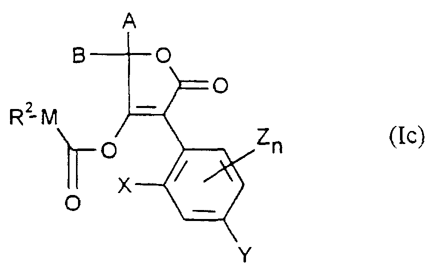 worin
A, B, E, M, X, Y, Z, R¹, R², und n die oben angegebenen Bedeutungen besitzen.

Aufgrund eines oder mehrerer Chiralitätszentren, fallen die Verbindungen der Formeln (Ia) bis (Ic) im allgemeinen als Stereoisomerengemisch an. Sie konnen sowohl in Form ihrer Diastereomerengemische als auch als reine Diastereomere oder Enantiomere verwendet werden.

Weiterhin wurde gefunden, daß man die neuen substituierten 3-Aryl-tetronsäure-Derivate der Formel (I) nach einem der im folgenden beschriebenen Verfahren erhält.
(A) Man erhält 3-Aryl-tetronsäuren der Formel (Ia) 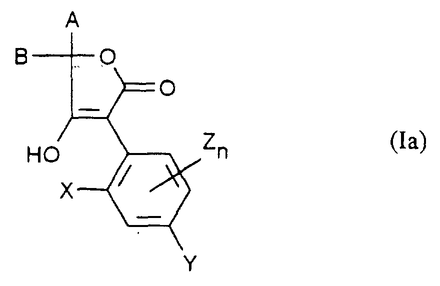 in welcher
   A, B, X, Y, Z und n die oben angegebene Bedeutung haben, wenn man
   Carbonsäureester der Formel (II) 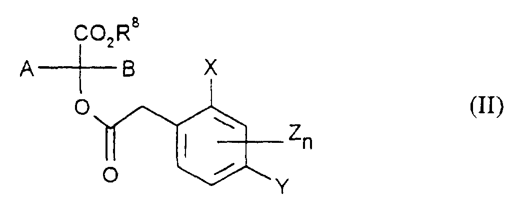 in welcher
   - A, B, X, Y, Z und n: die oben angegebene Bedeutung haben, und
   - R⁸: für Alkyl, bevorzugt C₁-C₈-Alkyl steht, in Gegenwart eines Verdünnungsmittels und in Gegenwart einer Base intramolekular kondensiert;
   und
(B) man erhält Verbindungen der Formel (Ib) 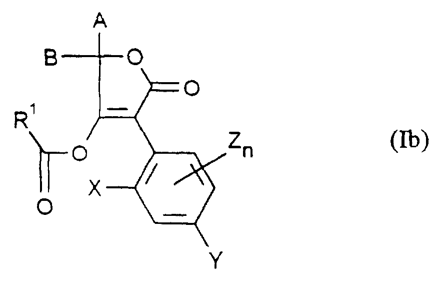 in welcher
   A, B, X, Y, Z, R¹ und n die oben angegebene Bedeutung haben, wenn man Verbindungen der Formel (Ia), 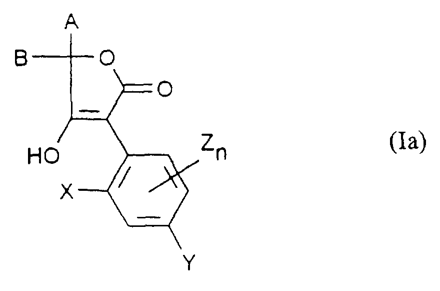 in welcher
   A, B, X, Y, Z und n die oben angegebene Bedeutung haben,
   α) mit Säurehalogeniden der Formel (III) 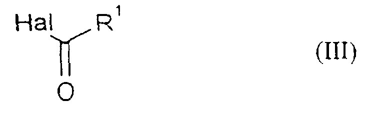 in welcher
      - R¹: die oben angegebene Bedeutung hat und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
      umsetzt;
   oder
(C) man erhält Verbindungen der Formel (Ic) 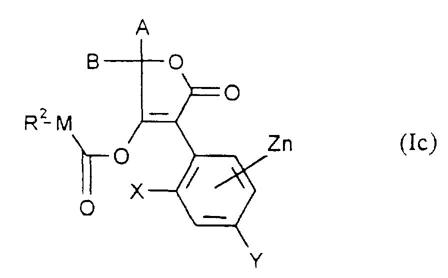 in welcher
   A, B, X, Y, Z, R² und n die oben angegebene Bedeutung haben,
   und
   - M: für Sauerstoff oder Schwefel steht,
   wenn man Verbindungen der Formel (Ia) 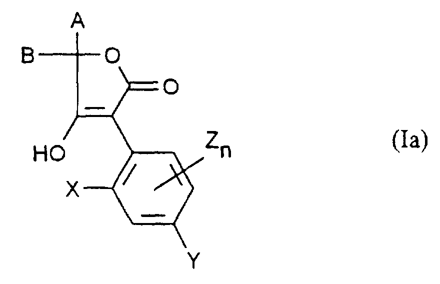 in welcher
   - A, B, X, Y, Z und n: die oben angegebene Bedeutung haben,
   mit Chlorameisensäureester oder Chlorameisensäurethiolester der Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt.

Weiterhin wurde gefunden, daß sich die neuen Verbindungen der Formel (I) durch hervorragende insektizide und akarizide Wirkungen auszeichnen. Bevorzugte Substituenten bzw. Bereiche der in den oben und nachstehend erwähnten Formeln aufgeführten Reste werden im folgenden erläutert.
- A und B: stehen bevorzugt für gegebenenfalls einfach bis vierfach, gleich oder verschieden durch Fluor, Chlor, C₁-C₆-Alkyl, C₃-C₆-Cycloalkyl, C₁-C₆-Halogenalkyl, C₁-C₄-Alkoxy, C₁-C₄-Alkylthio oder Phenyl substituiertes C₄-C₅-Alkandiyl, in welchem ein oder zwei nicht direkt benachbarte Kohlenstoffatome durch die Gruppe 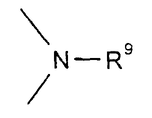 oder durch Sauerstoff oder durch Schwefel ersetzt ist.
- A und B: stehen besonders bevorzugt für gegebenenfalls einfach bis dreifach, gleich oder verschieden durch Fluor, Chlor, Methyl, Ethyl, n-Propyl, i-Propyl, n-Butyl, i-Butyl, sec-Butyl, tert.-Butyl, Cyclohexyl, Trifluormethyl, Methoxy, Methylthio oder Phenyl substituiertes C₄-C₅-Alkandiyl, in welchem ein Kohlenstoffatom durch die Gruppe 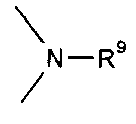 oder durch Sauerstoff oder durch Schwefel ersetzt ist.
- X: steht bevorzugt für C₁-C₄-Alkyl, Fluor, Chlor, Brom oder C₁-C₄-Alkoxy
- X: steht besonders bevorzugt für Methyl, Ethyl, n-Propyl, iso-Propyl, Fluor, Chlor, Brom, Methoxy oder Ethoxy.
- Y: steht bevorzugt für Wasserstoff, C₁-C₄-Alkyl, Fluor, Chlor, Brom, C₁-C₄-Alkoxy oder C₁-C₂-Halogenalkyl.
- Y: steht besonders bevorzugt für Wasserstoff, Methyl, Ethyl, n-Propyl, i-Propyl, n-Butyl, i-Butyl, tert.-Butyl, Fluor, Chlor, Brom, Methoxy, Ethoxy oder Trifluormethyl.
- Z: steht bevorzugt für C₁-C₄-Alkyl, Fluor, Chlor, Brom oder C₁-C₄-Alkoxy.
- Z: steht besonders bevorzugt für Methyl, Ethyl, Propyl, i-Propyl, n-Butyl, i-Butyl, tert.-Butyl, Fluor, Chlor, Brom, Methoxy oder Ethoxy.
- R¹: steht bevorzugt für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis dreifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach durch Fluor, Chlor, Brom oder C₁-C₄-Alkyl substituiertes Pyridyl, Thienyl, Furanyl, Pyrazolyl, Pyrimidyl oder Thiazolyl,
für gegebenenfalls einfach oder zweifach durch Fluor, Chlor, Brom oder C₁-C₄-Alkyl substituiertes Phenoxy-C₁-C₅-alkyl oder
für jeweils gegebenenfalls einfach oder zweifach durch Fluor, Chlor, Brom, Amino oder C₁-C₄-Alkyl substituiertes Pyrimidinyloxy-C₁-C₅-alkyl, Pyridinyloxy-C₁-C₅-alkyl oder Thiazolyloxy-C₁-C₅-alkyl.
- R²: steht bevorzugt für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis sechsfach durch Fluor, Chlor, C₁-C₃-Alkyl oder C₁-C₃-Alkoxy substituiertes C₃-C₇-Cycloalkyl oder
für gegebenenfalls einfach oder zweifach durch Fluor, Chlor, Brom, Nitro, C₁-C₄-Alkyl, C₁-C₃-Alkoxy oder C₁-C₃-Halogenalkyl substituiertes Phenyl oder Benzyl.
- R⁹: steht bevorzugt für Wasserstoff, Q, COQ oder CO₂Q,
wobei
Q für C₁-C₆-Alkyl, Phenyl oder Benzyl steht.
- n: steht bevorzugt für 0 oder 1.
- R¹: steht besonders bevorzugt für jeweils gegebenenfalls einfach bis dreifach durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, Propyl, i-Propyl, Butyl, i-Butyl, t-Butyl, Methoxy, Ethoxy, Propoxy, i-Propoxy, Butoxy, i-Butoxy, s-Butoxy oder t-Butoxy substituiertes C₃-C₆-Cycloalkyl, in welchem gegebenenfalls eine Methylengruppe durch Sauerstoff oder Schwefel ersetzt ist,
für gegebenenfalls einfach oder zweifach durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl oder Nitro substituiertes Phenyl,
für gegebenenfalls einfach oder zweifach durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl,
für jeweils gegebenenfalls einfach oder zweifach durch Fluor, Chlor, Brom, Methyl oder Ethyl substituiertes Thienyl, Furanyl oder Pyridyl,
für gegebenenfalls einfach oder zweifach durch Fluor, Chlor, Methyl oder Ethyl substituiertes Phenoxy-C₁-C₄-alkyl oder
für jeweils gegebenenfalls einfach oder zweifach durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht besonders bevorzugt für jeweils gegebenenfalls einfach bis dreifach durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls einfach bis dreifach durch Fluor, Chlor, Methyl, Ethyl, Propyl, iso-Propyl oder Methoxy substituiertes C₃-C₆-Cycloalkyl,
oder für jeweils gegebenenfalls einfach oder zweifach durch Fluor, Chlor, Nitro, Methyl, Ethyl, Propyl, i-Propyl, Methoxy, Ethoxy oder Trifluormethyl substituiertes Phenyl oder Benzyl.
- R⁹: steht besonders bevorzugt für Wasserstoff, Q, COQ oder CO₂Q, wobei
Q für C₁-C₄-Alkyl, Phenyl oder Benzyl steht.
- n: steht besonders bevorzugt für 0 oder 1.

In den angegebenen Definitionen können gesättigte oder ungesättigte Kohlenwasserstoffreste auch in Verbindung mit Heteroatomen, wie z.B. Alkoxy oder Alkylthio, soweit möglich, geradkettig oder verzweigt sein

Die gegebenenfalls mehrfach substituierten Reste können gleich oder verschieden durch die für diese Reste genannten Substituenten substituiert sein.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Eine bevorzugte Gruppe von Verbindungen sind diejenigen Verbindungen der Formel (I), in welchen n für die Zahl 1 steht und sich der Substituent Z in der 6-Stellung des Phenylrestes befindet.

Eine weitere bevorzugte Gruppe von Verbindungen sind diejenigen Verbindungen der Formel (I), in welchen gleichzeitig n für die Zahl 0 steht und Y nicht für Wasserstoff steht.

Eine außerdem bevorzugte Gruppe von Verbindungen sind diejenigen Verbindungen der Formel (I), in welcher gleichzeitig n für die Zahl 1 steht und Y für Wasserstoff steht.

Im einzelnen seien außer den bei den Herstellungsbeispielen genannten Verbindungen die in den folgenden Tabelle 1 bis 4 aufgeführten Verbindungen der Formel (Ia) genannt:

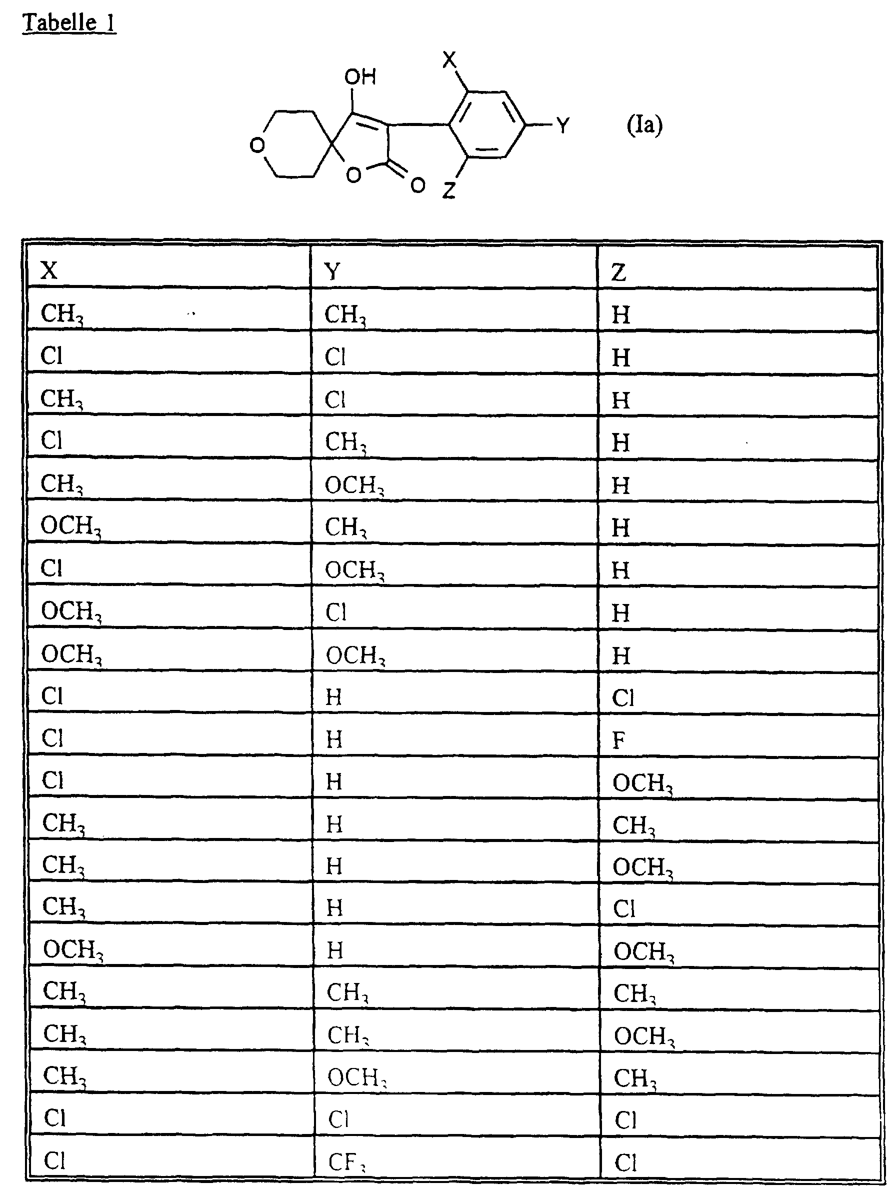

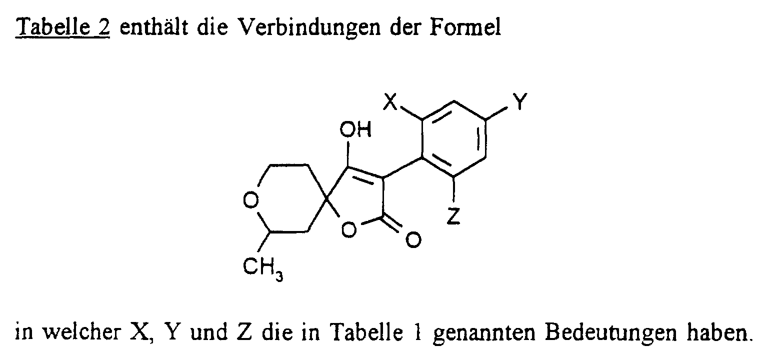

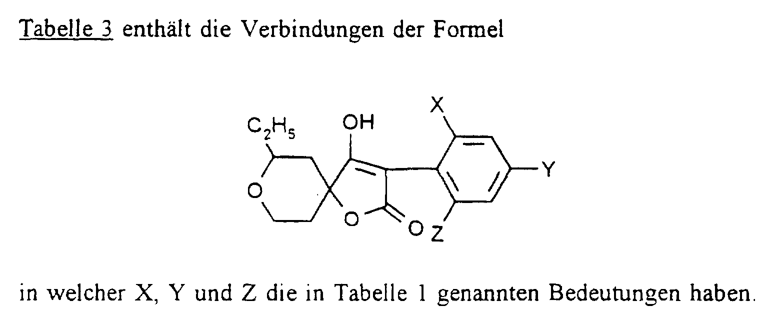

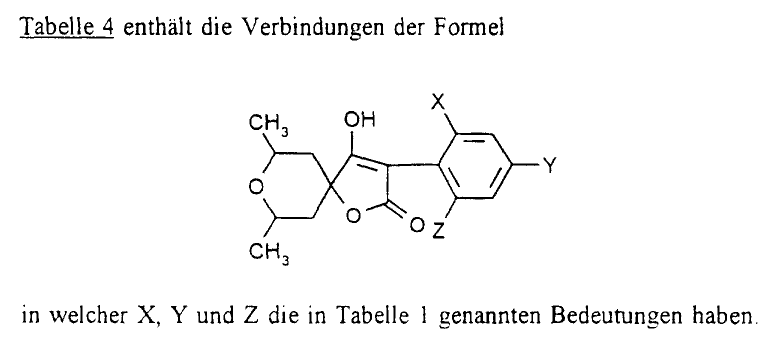

Verwendet man gemäß **Verfahren (A)** 1-(2,4-Dichlorphenylacetyloxy)-4-oxacyclohexan-carbonsäureethylester, so kann der Verlauf des erfindungsgemäßen Verfahrens durch folgendes Reaktionsschema wiedergegeben werden:

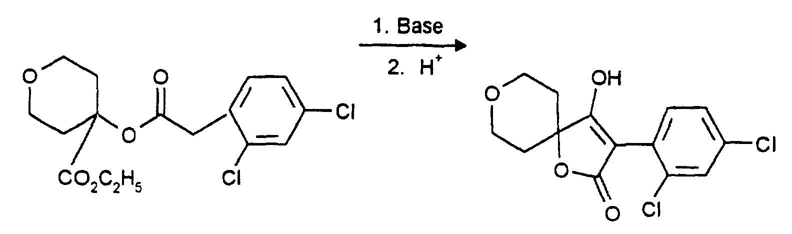

Verwendet man gemäß **Verfahren (B) (Variante α)** 3-(2,4,6-Trimethylphenyl)-5,5-(ethylenoxaethylen)-tetronsäure und Pivaloylchlorid als Ausgangsstoffe, so kann der Verlauf des erfindungsgemäßen Verfahrens durch folgendes Reaktionsschema wiedergegeben werden:

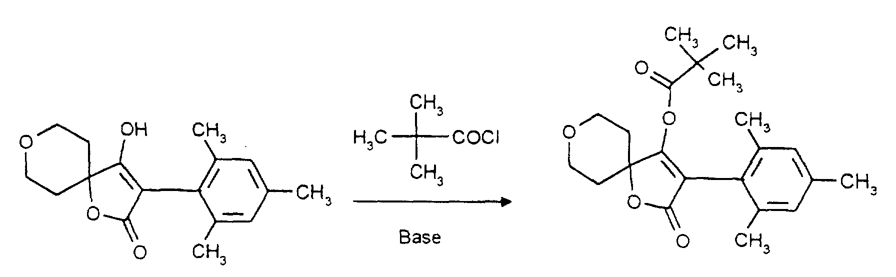

Verwendet man gemäß **Verfahren B (Variante β)** 3-(2,4,6-Trimethylphenyl)-5,5-ethylenoxamethylen-tetronsäure und Acetanhydrid als Ausgangsverbindungen, so kann der Verlauf des erfindungsgemäßen Verfahrens durch folgendes Reaktionsschema wiedergegeben werden:

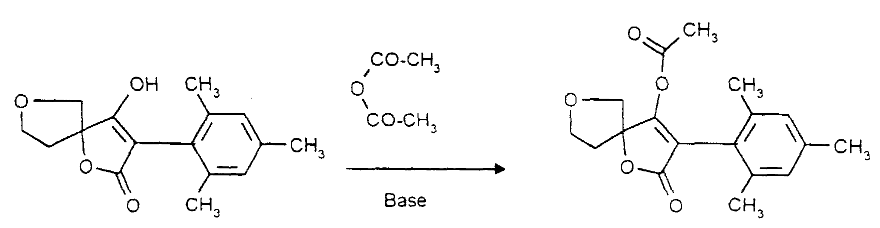

Verwendet man gemäß **Verfahren (C)** 3-(2,4,6-Trimethylphenyl)-5,5-ethylenoxamethylen-tetronsäure und Chlorameisensäureethoxyethylester als Ausgangsverbindungen, so kann der Verlauf des erfindungsgemäßen Verfahrens durch folgendes Reaktionsschema wiedergegeben werden:

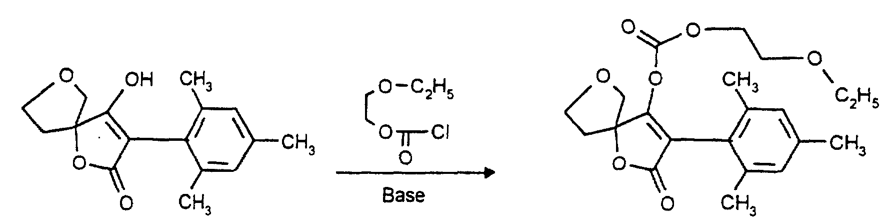

Die bei dem obigen Verfahren (A) als Ausgangsstoffe benötigten Verbindungen der Formel (II) 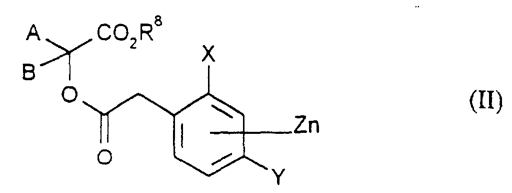 in welcher
- A, B, X, Y, Z, n und R⁸: die oben angegebene Bedeutung haben,
sind neu.

Den Zwischenverbindungen der Formel (II) strukturell verwandte spezifiche Verbindungen sind beispielsweise aus EP 0 528 156 A, EP 0 647 637 A, WO 95 01971 A, EP 0 668 267 A, DE 92 589 A, J. GEN. CHEM. U.S.S.R., 26 (10), 10/1956, S. 3891-3898, Nazarov et al. und ibid, 30 (12), 12/1960, S. 3883-3887, Unkovskii et al. bekannt.

Man erhält die Verbindungen der Formel (II) beispielsweise, wenn man
2-Hydroxycarbonsäure-Derivate der Formel (XIV), 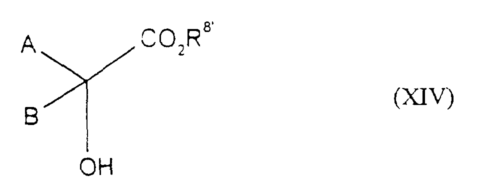 in welcher
- R^{8'}: für Wasserstoff (XIVa) oder Alkyl (bevorzugt C₁-C₈-Alkyl) (XIVb) steht und
A und B die oben angegebene Bedeutung haben,
mit Phenylessigsäurehalogeniden der Formel (XV) 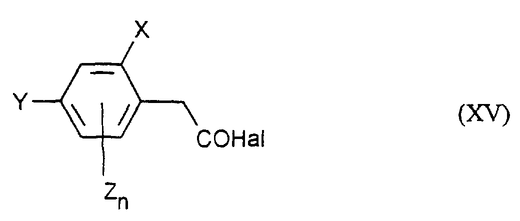 in welcher
- X, Y, Z und n: die oben angegebene Bedeutung haben und
- Hal: für Chlor oder Brom steht,
acyliert (Chem. Reviews 52, 237-416 (1953)); und gegebenenfalls die dabei für R^{8'} = Wasserstoff gebildeten Verbindungen der Formel (IIa) 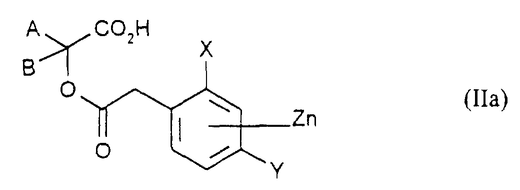 in welcher
- A, B, X, Y, Z und n: die oben angegebene Bedeutung haben,
verestert (Chem. Ind. (London) 1568 (1968)).

Die Verbindungen der Formel (XIV) sind teilweise bekannt und/oder lassen sich nach bekannten Verfahren herstellen, beispielsweise aus den Cyanhydrinen der Formel (XVI) 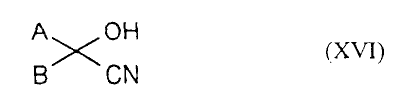 in welcher
A und B die oben angegebene Bedeutung haben,
[s. z.B. Nasarow, Unkowskii, Zh. Obshch. Khim. 26, 3486 (1956) oder Bennett, Waddington, J. Chem. Soc. 2831 (1929)].

Die Verbindungen der Formel (XVI) sind teilweise bekannt und/oder lassen sich nach bekannten Verfahren herstellen (z.B. Eichen, Fritz; Schmidt, Michal; Buchborn, Helga; Arch. Pharm. 320, 348-61, 1987; Sargsyan, M.S, Ukrtumyan, S.A; Gevorkyan A.A, Arm. Khim. Zh 38 494-8, 1985).

Die Phenylessigsäurehalogenide der Formel (XV) sind bekannt und/oder lassen sich nach bekannten Verfahren herstellen [s. z.B. Lutz, Hinkley, J. Amer. Chem. Soc. 72, 4091 (1950), Harispe, Ann. Chim. (Paris) 11, 6, S. 249, 282, 283 (1936)].

Die zur Durchführung der erfindungsgemäßen Verfahren (B), (C) und (D) außerdem als Ausgangsstoffe benötigten Säurehalogenide der Formel (III), Carbonsäureanhydride der Formel (IV), Chlorameisensäureester oder Chlorameisensäurethioester der Formel (V) sind allgemein bekannte Verbindungen der organischen bzw. anorganischen Chemie.

Das Verfahren (A) ist dadurch gekennzeichnet, daß man Verbindungen der Formel (II) in welcher A, B, X, Y, Z, n und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide oder -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, ®Adogen 464 (Methyltrialkyl(C₈-C₁₀)ammoniumchlorid) oder TDA 1 (Tris-(methoxyethzoxyethyl)-amin) eingesetzt werden können.

Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -30°C und 250°C, vorzugsweise zwischen 0°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in molaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (la) mit Carbonsäurehalogeniden der Formel (III) umsetzt.

Als Verdünnungsmittel können bei dem erfindungsgemäßen Verfahren (Bα) bei alle gegenüber den Säurehalogeniden ausreichend inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Die Reaktionstemperaturen können auch bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Bei dem erfindungsgemäßen Verfahren (Bβ) können als Verdunnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (Ia) und das Carbonsäureanhydrid der Formel (IV) im allgemeinen in angenähert äquivalenten Mengen eingesetzt. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethylanilin. ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid

Als Verdünnungsmittel können bei dem erfindungsgemäßen Verfahren (C) alle gegenüber den Verbindungen der Formel (V) ausreichend inerten Solventien eingesetzt werden. Vorzugsweise verwendbar sind Kohlenwasserstoffe, wie Benzin, Benzol, Toluol, Xylol und Tetralin. ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der Chlorameisensäureester bzw. Chlorameisensäurethiolester der Formel (V) im allgemeinen in angenähert äquivalenten Mengen eingesetzt. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Die Wirkstoffe eignen sich zur Bekampfung von tierischen Schädlingen, insbesondere Insekten, Spinnentieren und Nematoden, die in der Landwirtschaft, in Forsten, im Vorrats- und Materialschutz sowie auf dem Hygienesektor vorkommen Sie können vorzugsweise als Pflanzenschutzmittel eingesetzt werden. Sie sind gegen normal sensible und resistente Arten sowie gegen alle oder einzelne Entwicklungsstadien wirksam Zu den oben erwähnten Schädlingen gehören.

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis. Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Verbindungen der Formel (I) zeichnen sich durch eine hohe insektizide und akarizide Wirksamkeit aus. Sie sind auch bodeninsektizid wirksam.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) oder zur Bekämpfung von pflanzenschädigenden Milben, wie beispielsweise gegen die gemeine Spinnmilbe oder die Bohnenspinnmilbe (Tetranychus urticae) einsetzen.

Die Wirkstoffe der Formel (I) weisen auch eine gewisse herbizide Wirkung auf.

Die Wirkstoffe können in die üblichen Formulierungen überführt werden, wie Losungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflachenaktiven Mitteln, also Emulgiermitteln und/oder Dispergiermitteln und/oder schaumerzeugenden Mitteln.

Im Falle der Benutzung von Wasser als Streckmittel konnen z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb-stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat, 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium. Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxichlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivemectin, Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansauren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinarmedizinischen Sektor gegen tierische Parasiten (Ektoparasiten) wie Schildzecken, Lederzecken, Raudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus, Lucilia cuprina und Musca domestica.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbandern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfahige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel 1 eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticomis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie
Sirex juvencus, Urocerus gigas, Urocerus gigas taignus, Urocerus augur.

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder olartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on,
sein.
Die Herstellung und die Verwendung der erfindungsgemäßen Wirkstoffe gehen aus den nachfolgenden Beispielen hervor.

### Herstellungsbeispiele

### Beispiel (Ia-1)

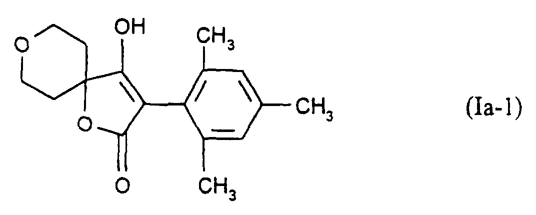

67,3 g (0,60 Mol) Kalium-tert.-butylat werden in 400 ml abs. Dimethylformamid (DMF) vorgelegt, bei 0 bis 10°C eine Lösung von 133,6 g (0,40 Mol) 1-(2,4,6-Trimethylphenylacetyl-oxy)-4-oxa-cyclohexan-carbonsäure-ethylester zugetropft und über Nacht bei Raumtemperatur gerührt.

Zur Aufarbeitung tropft man das Reaktionsgemisch langsam in 2 l eisgekühlte 1N Salzsäure ein, saugt den Niederschlag ab, wäscht gut mit Wasser nach und trocknet das Produkt im Vakuumtrockenschrank.

Zur Reinigung wird das Rohprodukt mit n-Hexan ausgekocht und erneut abgesaugt und getrocknet.

Ausbeute: 91,6 g (79 % der Theorie) eines Feststoffes; Fp.: 224-226°C.

Analog bzw. gemäß den allgemeinen Angaben zur Herstellung werden die folgenden Verbindungen der Formel (Ia) erhalten:
(In den Tabellen werden folgende Abkürzungen verwendet: Me = Methyl, Et = Ethyl, Pr = Propyl, Bu = Butyl, Ph = Phenyl)

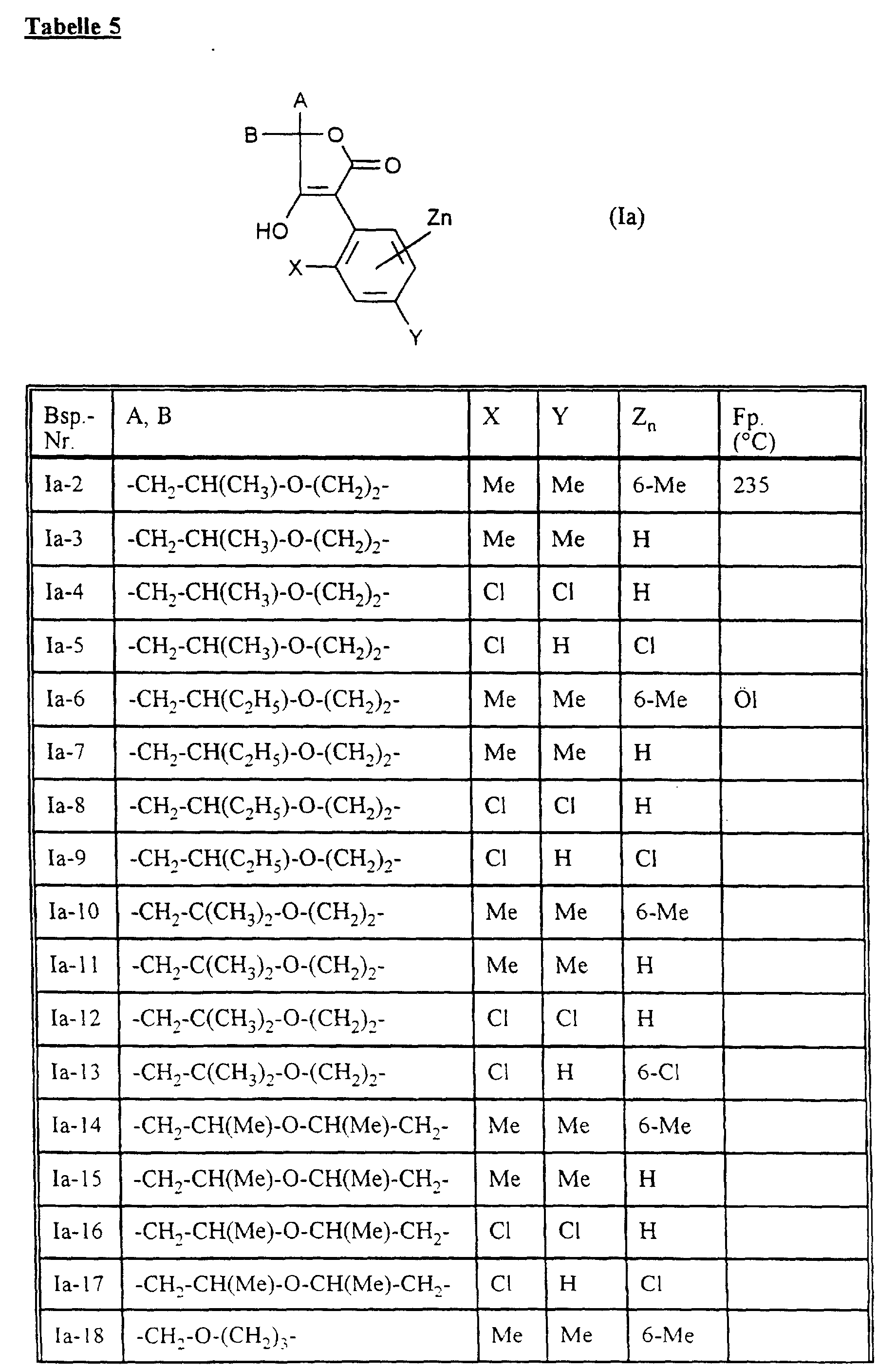

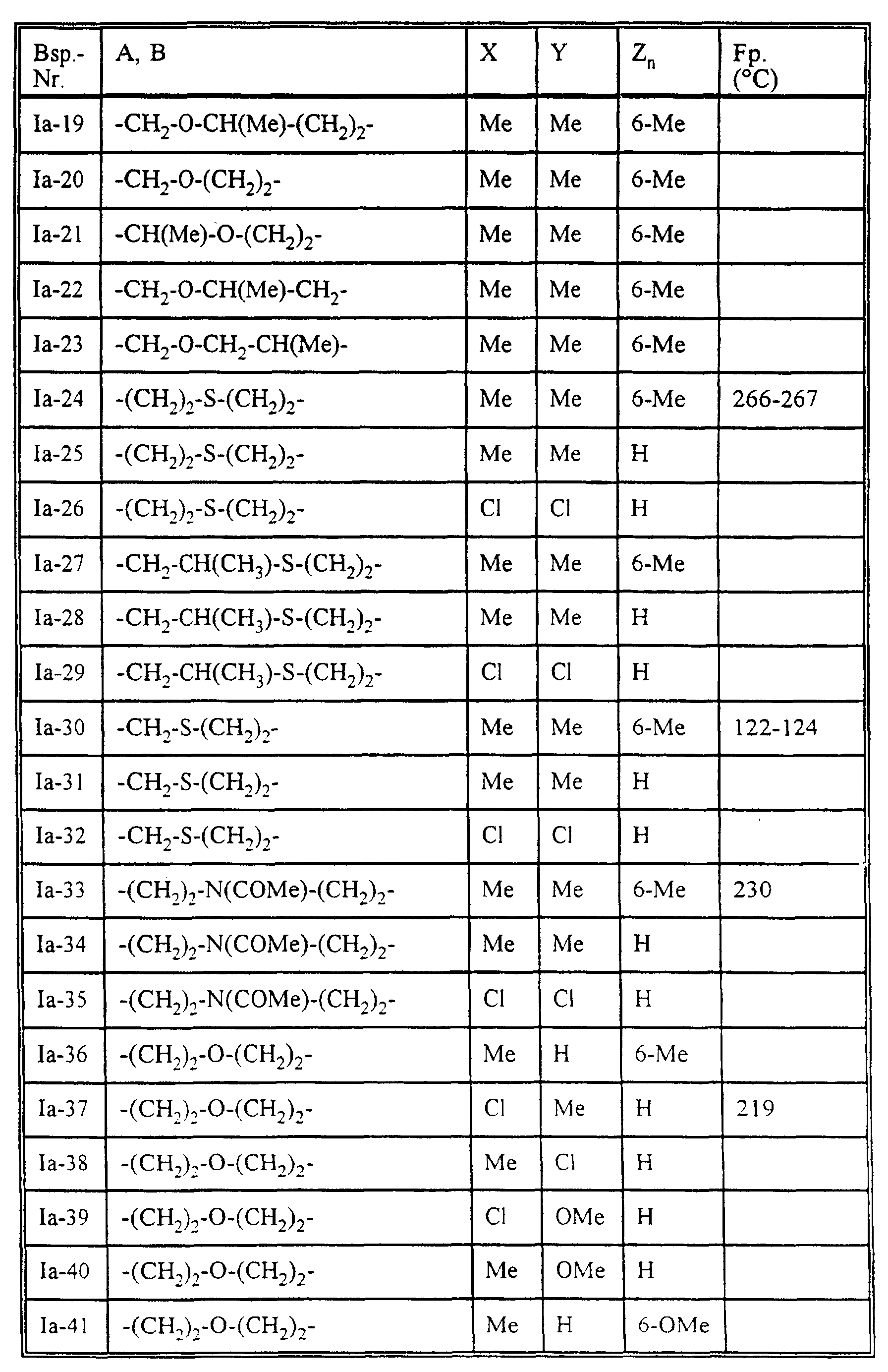

### Beispiel (Ib-1)

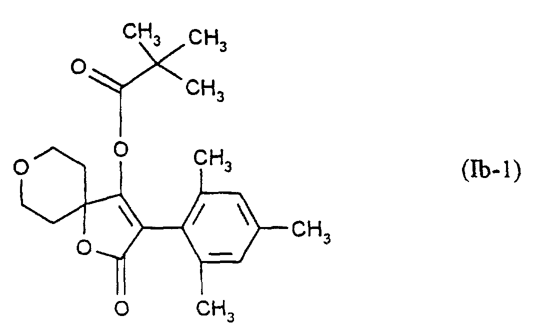

28,8 g (0,10 Mol) 3-(2,4,6-Trimethylphenyl)-5,5-ethylen-oxa-ethylentetronsäure gemäß Bsp. (Ia-1) werden in 400 ml abs. Methylenchlorid vorgelegt, mit 15,2 g (0,15 Mol) Triethylamin versetzt, bei 0 bis 10°C eine Lösung von 15,7 g (0,13 Mol) Pivaloylchlorid zugetropft und einige Stunden bei Raumtemperatur gerührt.

Zur Aufarbeitung wäscht man das Reaktionsgemisch nacheinander mit 10 %iger Citronensäure, Natriumhydrogencarbonatlösung und Wasser, trocknet die organische Phase über Natriumsulfat und dampft ein.

Zur Reinigung verrührt man das Rohprodukt mit Petrolether und saugt ab.

Ausbeute: 29,4 g (78 % der Theorie) eines Feststoffes; Fp.: 119-120°C.

Analog bzw. gemäß den allgemeinen Angaben zur Herstellung werden die folgenden Verbindungen der Formeln (Ib) bis (Ig) erhalten:

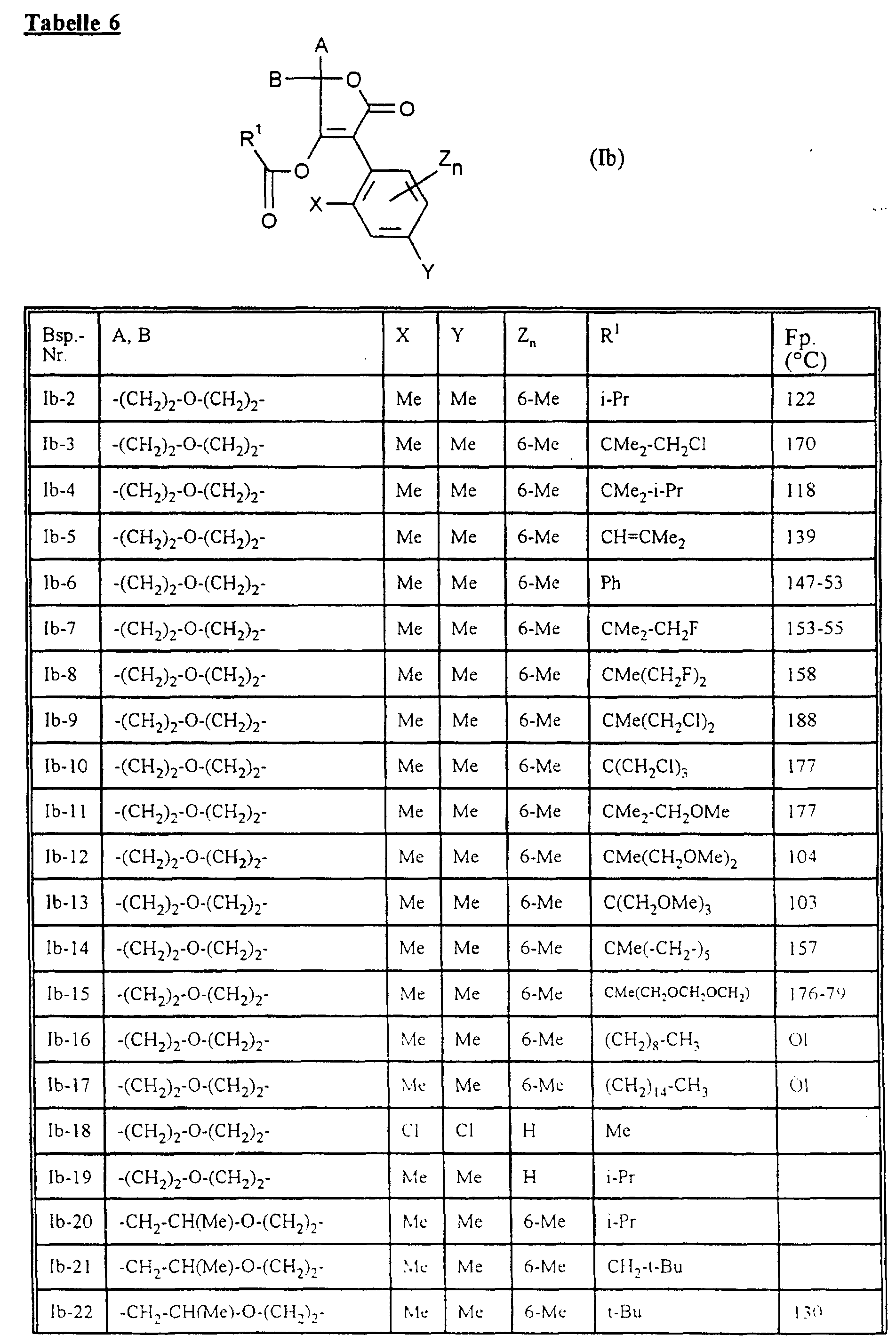

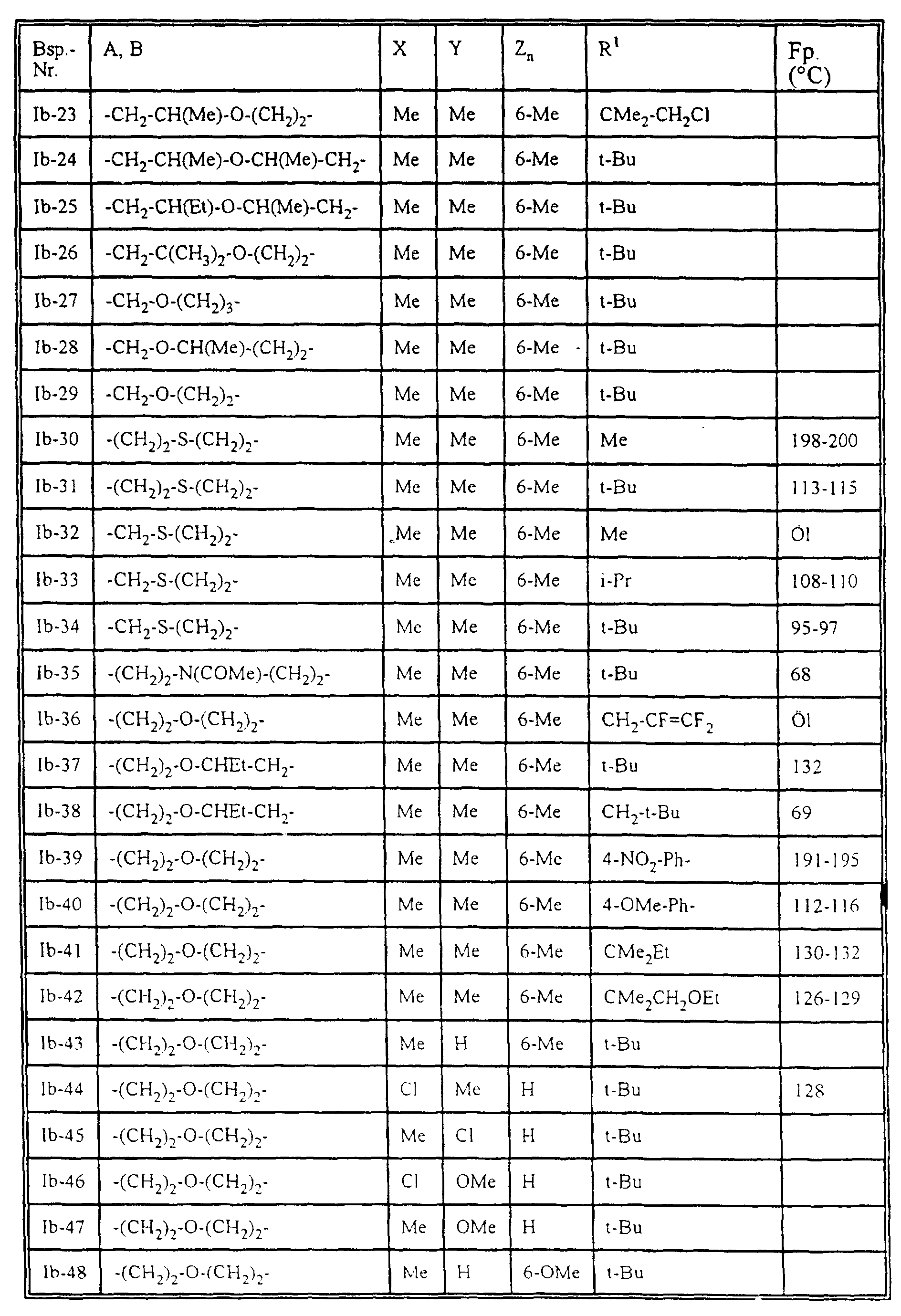

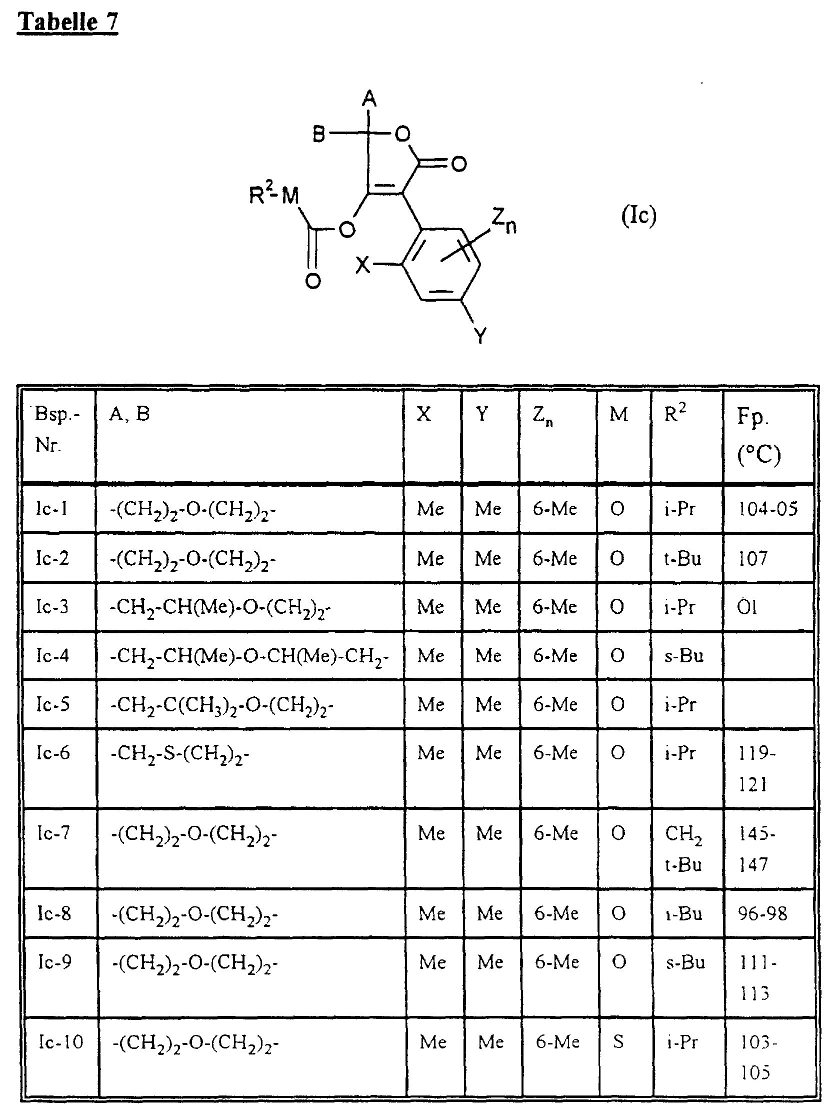

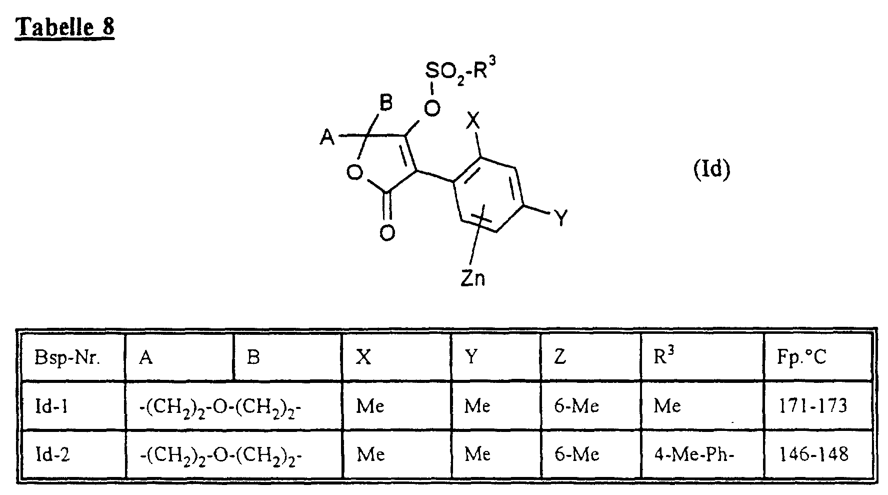

### Beispiel für die Herstellung eines Zwischenprodukts der Formel (II):

### Beispiel (II-1):

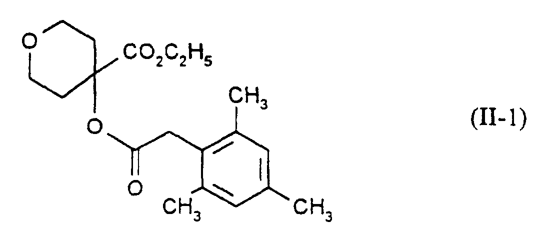

31,3 g (0,18 Mol) 1-Hydroxy-4-oxa-cyclohexancarbonsäureethylester werden in 150 ml Methylenchlorid vorgelegt, 21,9 g (0,216 Mol) Triethylamin zugesetzt, eine Lösung von 38,9 g (0,20 Mol) Mesitylessigsäurechlorid in 50 ml Methylenchlorid bei 0 bis 10°C zugetropft und über Nacht bei Raumtemperatur gerührt.

Zur Aufarbeitung wäscht man das Reaktionsgemisch nacheinander mit 10 %iger Citronensäure, Natriumhydrogencarbonatlösung und Wasser, trocknet die organische Phase über Magnesiumsulfat und dampft ein.

Ausbeute: 60,6 g eines Öls (quantitativ).

Analog bzw. gemäß den allgemeinen Angaben zur Herstellung werden die folgenden Verbindungen der Formel (II) erhalten:

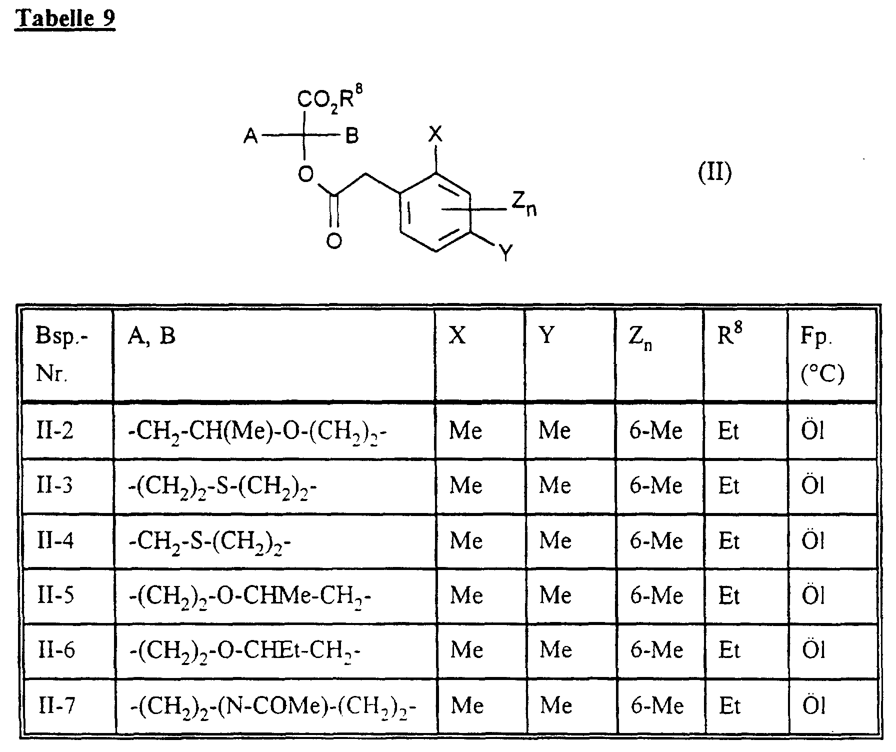

### Beispiel (XIV-1)

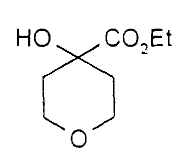

Eine Mischung aus 130 g (ca. 1 mol) 4-Hydroxy-tetrahydropyran-4-carbonsäurenitril und 1050 ml trockenem Ethanol wird bei -20°C bis 0°C mit Chlorwasserstoffsäure gesättigt. Man läßt auf Raumtemperatur kommen, entfernt überschussige HCl, engt im Vakuum ein, versetzt mit 1,5 l Wasser und rührt 3 Stunden bei Raumtemperatur. Man filtriert und extrahiert 2 mal mit Methylenchlorid. Nach dem Entfernen des Lösungsmittels erhalt man 118 g (61 % der Theorie) 4-Hydroxy-tetrahydropyran-4-carbonsaureethylester vom KP_{0.1} 65°C.

In den Anwendungsbeispielen wurden die folgenden, aus dem Stand der Technik bekannten Vergleichsverbindungen eingesetzt: 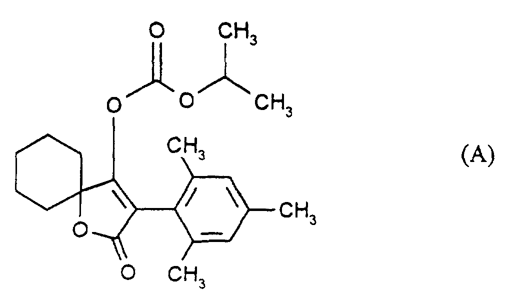 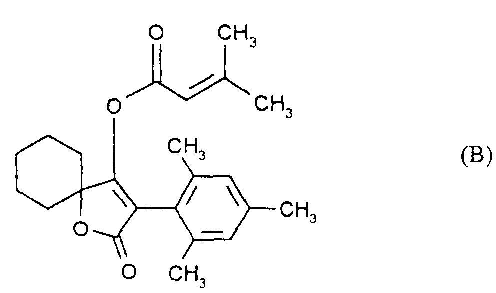 (alle bekannt aus EP-528 156)

### Beispiel A

| Myzus-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattlaus abgetötet wurde.

Bei diesem Test bewirkten z.B. die Verbindungen gemäß den Herstellungsbeispielen Ia-1, Ib-1, Ib-12, Ib-15 und Ic-1 bei einer beispielhaften Wirkstoffkonzentration von 0,1 % eine Abtötung von mindestens 95 % nach 6 Tagen, während die aus dem Stand der Technik bekannte Verbindung (B) bei einer Wirkstoffkonzentration von 0,1 % eine Abtötung von nur 70 % bewirkte.

### Beispiel B

| Grenzkonzentrations-Test / Wurzelsystemische WSirkung | |
|---|---|
| Testinsekt | Aphis fabae |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Dicken Bohnen (Vicia faba). Der Wirkstoff kann so von den Pflanzenwurzeln aus dem Boden aufgenommen und in die Blätter transportiert werden.

Für den Nachweis des wurzelsystemischen Effektes werden nach 8 Tagen die Blätter mit den oben genannten Testtieren besetzt. Nach weiteren 6 Tagen erfolgt die Auswertung durch Zählen oder Schätzen der toten Tiere. Aus den Abtötungszahlen wird die wurzelsystemische Wirkung des Wirkstoffes abgeleitet. Sie ist 100 %, wenn alle Testtiere abgetötet sind und 0 %, wenn noch genau so viele Testinsekten leben wie bei der unbehandelten Kontrolle.

In diesem Test zeigten z.B. die Verbindungen gemäß den Herstellungsbeispielen Ia-1, Ib-2, Ib-5, Ib-7, Ib-8, Ib-12, Ib-15, Ib-16, Ib-17 und Ic-2 bei einer beispielhaften Wirkstoffkonzentration von 200 ppm eine Wirkung von 100 %.

### Beispiel C

| Nephotettix - Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100%, daß alle Zikaden abgetötet wurden; 0 % bedeutet, daß keine Zikade abgetötet wurde.

Bei diesem Test bewirkten z.B. die Verbindungen gemäß den Herstellungsbeispielen Ia-1, Ib-1, Ib-16, Ic-1 und Ic-3 bei einer beispielhaften Wirkstoffkonzentration von 0,01 % eine Abtötung von 100 % nach 6 Tagen, während die bekannte Verbindung (A) keine Abtötung bewirkte.

### Beispiel D

| Tetranychus - Test, (OP-resistent) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0 % bedeutet, daß keine Spinnmilbe abgetötet wurde.

Bei diesem Test bewirkten z.B die Verbindungen gemäß den Herstellungsbeispielen Ia-1, Ia-24, Ib-1, Ib-2, Ib-16, Ib-31 und Ic-1 bei einer beispielhaften Wirkstoffkonzentration von 0,02 % eine Abtötung von mindestens 95 % nach 7 Tagen und die Verbindungen gemäß den Herstellungsbeispielen Ib-5, Ib-7, Ib-11 und Ib-22 bei einer beispielhaften Wirkstoffkonzentration von 0,1 % eine Abtötung von mindestens 98 % nach 7 Tagen.

### Beispiel E

| Panonychus - Test | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Ca. 30 cm hohe Pflaumenbäumchen (Prunus domestica), die stark von allen Entwicklungsstadien der Obstbaumspinnmilbe (Panonychus ulmi) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilbe abgetötet wurde.

Bei diesem Test bewirkten z.B. die Verbindungen gemäß den Herstellungsbeispielen Ia-1, Ia-24, Ib-1, Ib-2, Ib-16, Ib-31 und Ic-1 bei einer beispielhaften Wirkstoffkonzentration von 0,02 % eine Abtötung von mindestens 95 % nach 7 Tagen.

### Beispiel F

| Test mit Fliegenlarven / Entwicklungshemmende Wirkung | |
|---|---|
| Testtiere | Alle larvalen Stadien von Lucilia cuprina (OP-resistent) |
| | [Puppen und Adulte (ohne Kontakt zum Wirkstoff)] |
| Lösungsmittel | 35 Gewichtsteile Ethylenglykolmonomethylether |
| | 35 Gewichtsteile Nonylphenolglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larziver Effekt), bzw. die Hemmung des Adultschlupfes aus den Puppen bzw die Hemmung der Puppenbildung. Als Kriterium für die in-vitro-Wirkung einer Substanz gilt die Hemmung der Flohentwicklung, bzw ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulte Fliegen geschlüpft sind.

In diesem Test zeigten z.B. die Verbindungen gemäß den Herstellungsbeispielen Ia-2, Ia-24, Ib-4, Ib-15, Ib-22, Ib-24 und Ib-30 bei einer beispielhaften Wirkstoffkonzentration von 1000 ppm eine Wirkung von jeweils 100 %.

## Patentansprüche

1. Verbindungen der Formel (I) 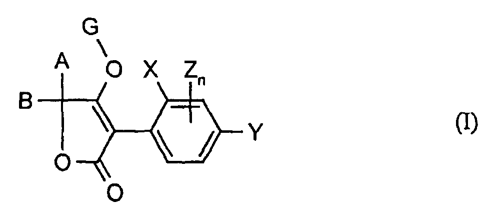 in welcher
A und B für gegebenenfalls einfach oder mehrfach, gleich oder verschieden durch Halogen, C₁-C₈-Alkyl, C₃-C₈-Cycloalkyl, C₁-C₈-Halogenalkyl, C₁-C₆-Alkoxy, C₁-C₆-Alkylthio oder Phenyl substituiertes C₄-C₆-Alkandiyl stehen, in welchem ein oder zwei nicht direkt benachbarte Kohlenstoffatome durch die Gruppe 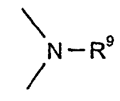 und/oder Sauerstoff und/oder Schwefel ersetzt sind,
X für C₁-C₆-Alkyl, Halogen oder C₁-C₆-Alkoxy steht,
Y für Wasserstoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
G für Wasserstoff (a) oder für eine der Gruppen 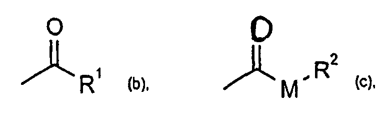 steht, in welchen
M für Sauerstoff oder Schwefel steht,
R¹ für jeweils gegebenenfalls einfach oder mehrfach durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₂-C₈-alkyl oder gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls einfach bis fünffach durch Halogen, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls einfach bis fünffach durch Halogen, C₁-C₆-Alkyl, C i-C₆-Alkoxy, C₁-C₆-Halogenalkyl oder C₁-C₆-Halogenalkoxy substituiertes Phenyl-C₁-C₆-alkyl,
für jeweils gegebenenfalls einfach oder zweifach durch Halogen oder C₁-C₆-Alkyl substituiertes Pyridyl, Thienyl, Furanyl, Pyrazolyl, Pyrimidyl oder Thiazolyl,
für gegebenenfalls einfach bis dreifach durch Halogen oder C₁-C₆-Alkyl substituiertes Phenoxy-C₁-C₆-alkyl oder
für jeweils gegebenenfalls einfach oder zweifach durch Halogen, Amino oder C₁-C₆-Alkyl substituiertes Pyridinyloxy-C₁-C₆-alkyl, Pyrimidinyloxy-C₁-C₆-alkyl oder Thiazolyloxy-C₁-C₆-alkyl steht,
R² für jeweils gegebenenfalls einfach oder mehrfach durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls einfach oder mehrfach durch Halogen, C₁-C₄-Alkyl oder C₁-C₄-Alkoxy substituiertes C₃-C₈-Cycloalkyl oder
für jeweils gegebenenfalls einfach bis dreifach durch Halogen, Nitro, C₁-C₆-Alkyl, C₁-C₆-Alkoxy oder C₁-C₆-Halogenalkyl substituiertes Phenyl oder Benzyl steht,
R⁹ für Wasserstoff, Q, COQ oder CO₂Q steht,
wobei
Q die oben für R² genannten Bedeutungen annehmen kann und
n 0, 1 oder 2 steht.

2. Verbindungen der Formel (I) gemäß Anspruch 1, in welcher
A und B für gegebenenfalls einfach bis vierfach, gleich oder verschieden durch Fluor, Chlor, C₁-C₆-Alkyl, C₃-C₆-Cycloalkyl, C₁-C₆-Halogenalkyl, C₁-C-₄-Alkoxy, C₁-C₄-Alkylthio oder Phenyl substituiertes C₄-C₅-Alkandiyl stehen, in welchem ein oder zwei nicht direkt benachbarte Kohlenstoffatome durch die Gruppe 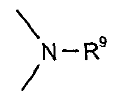 oder durch Sauerstoff oder durch Schwefel ersetzt ist.
X für C₁-C₄-Alkyl, Fluor, Chlor, Brom oder C₁-C₄-Alkoxy steht,
Y für Wasserstoff, C₁-C₄-Alkyl, Fluor, Chlor, Brom, C₁-C₄-Alkoxy oder C₁-C₂-Halogenalkyl steht,
Z für C₁-C₄-Alkyl, Fluor, Chlor, Brom oder C₁-C₄-Alkoxy steht,
G für Wasserstoff (a) oder für eine der Gruppen 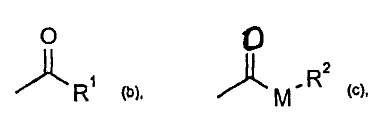 steht, in welchen
M für Sauerstoff oder Schwefel steht,
R¹ für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis dreifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach durch Fluor, Chlor, Brom oder C₁-C₄-Alkyl substituiertes Pyridyl, Thienyl, Furanyl, Pyrazolyl, Pyrimidyl oder Thiazolyl,
für gegebenenfalls einfach oder zweifach durch Fluor, Chlor, Brom oder C₁-C₄-Alkyl substituiertes Phenoxy-C₁-C₅-alkyl oder
für jeweils gegebenenfalls einfach oder zweifach durch Fluor, Chlor, Brom, Amino oder C₁-C₄-Alkyl substituiertes Pyrimidinyloxy-C₁-C₅-alkyl, Pyridinyloxy-C₁-C₅-alkyl oder Thiazolyloxy-C₁-C₅-alkyl steht,
R² für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis sechsfach durch Fluor, Chlor, C₁-C₃-Alkyl oder C₁-C₃-Alkoxy substituiertes C₃-C₇-Cycloalkyl oder
für gegebenenfalls einfach oder zweifach durch Fluor, Chlor, Brom, Nitro, C₁-C₄-Alkyl, C₁-C₃-Alkoxy oder C₁-C₃-Halogenalkyl substituiertes Phenyl oder Benzyl steht,
R⁹ für Wasserstoff, Q, COQ oder CO₂Q steht,
wobei
Q für C₁-C₆-Alkyl, Phenyl oder Benzyl steht und
n für 0 oder 1 steht.

3. Verbindungen der Formel (I) gemäß Anspruch 1, in welcher
A und B für gegebenenfalls einfach bis dreifach, gleich oder verschieden durch Fluor, Chlor, Methyl, Ethyl, n-Propyl, i-Propyl, n-Butyl, i-Butyl, sec.-Butyl, tert.-Butyl, Cyclohexyl, Trifluormethyl, Methoxy, Methylthio oder Phenyl substituiertes C₄-C₅-Alkandiyl stehen, in welchem ein Kohlenstoffatom durch die Gruppe 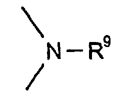 oder durch Sauerstoff oder durch Schwefel ersetzt ist,
X für Methyl, Ethyl, n-Propyl, iso-Propyl, Fluor, Chlor, Brom, Methoxy oder Ethoxy steht,
Y für Wasserstoff, Methyl, Ethyl, n-Propyl, i-Propyl, n-Butyl, i-Butyl, tert.-Butyl, Fluor, Chlor, Brom, Methoxy, Ethoxy oder Trifluormethyl steht,
Z für Methyl, Ethyl, Propyl, i-Propyl, n-Butyl, i-Butyl, tert.-Butyl, Fluor, Chlor, Brom, Methoxy oder Ethoxy steht,
G für Wasserstoff (a) oder für eine der Gruppen 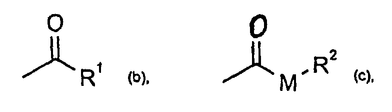 steht, in welchen
M für Sauerstoff oder Schwefel steht,
R¹ für jeweils gegebenenfalls einfach bis dreifach durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, Propyl, i-Propyl, Butyl, i-Butyl, t-Butyl, Methoxy, Ethoxy, Propoxy, i-Propoxy, Butoxy, i-Butoxy, s-Butoxy oder t-Butoxy substituiertes C₃-C₆-Cycloalkyl, in welchem gegebenenfalls eine Methylengruppe durch Sauerstoff oder Schwefel ersetzt ist,
für gegebenenfalls einfach oder zweifach durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl oder Nitro substituiertes Phenyl,
für gegebenenfalls einfach oder zweifach durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl,
für jeweils gegebenenfalls einfach oder zweifach durch Fluor, Chlor, Brom, Methyl oder Ethyl substituiertes Thienyl, Furanyl oder Pyridyl,
für gegebenenfalls einfach oder zweifach durch Fluor, Chlor, Methyl oder Ethyl substituiertes Phenoxy-C₁-C₄-alkyl oder
für jeweils gegebenenfalls einfach oder zweifach durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls einfach bis dreifach durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls einfach bis dreifach durch Fluor, Chlor, Methyl, Ethyl, Propyl, iso-Propyl oder Methoxy substituiertes C₃-C₆-Cycloalkyl,
oder für jeweils gegebenenfalls einfach oder zweifach durch Fluor, Chlor, Nitro, Methyl, Ethyl, Propyl, i-Propyl, Methoxy, Ethoxy oder Trifluormethyl substituiertes Phenyl oder Benzyl steht,
R⁹ für Wasserstoff, Q, COQ oder CO₂Q steht,
wobei
Q für C₁-C₄-Alkyl, Phenyl oder Benzyl steht und
n für 0 oder 1 steht.

4. Verfahren zur Herstellung von Verbindungen der Formel (I) gemäß Anspruch 1, **dadurch gekennzeichnet, dass** man
(A) zum Erhalt von Verbindungen der Formel (Ia) 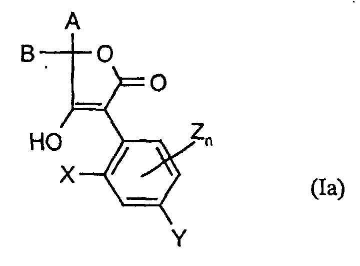 in welcher
A, B, X, Y, Z und n die in Anspruch 1 angegebene Bedeutung haben,
Carbonsäureester der Formel (II) 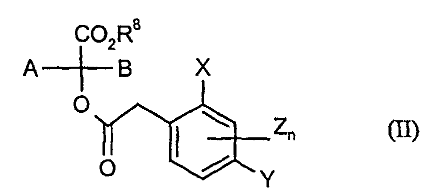 in welcher
A, B, X, Y, Z und n die oben angegebene Bedeutung haben, und
R⁸ für Alkyl steht,
in Gegenwart eines Verdünnungsmittels und in Gegenwart einer Base intramolekular kondensiert;
oder
(B) zum Erhalt von Verbindungen der Formel (Ib) 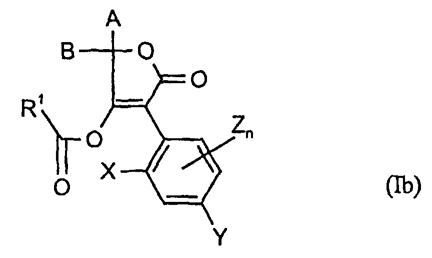 in welcher
A, B, X, Y, Z, R¹ und n die in Anspruch 1 angegebene Bedeutung haben,
Verbindungen der Formel (Ia), 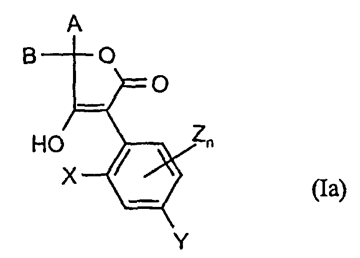 in welcher
A, B, X, Y, Z und n die oben angegebene Bedeutung haben,
α) mit Säurehalogeniden der Formel (III) 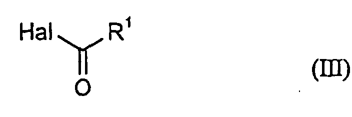 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) zum Erhalt von Verbindungen der Formel (Ic) 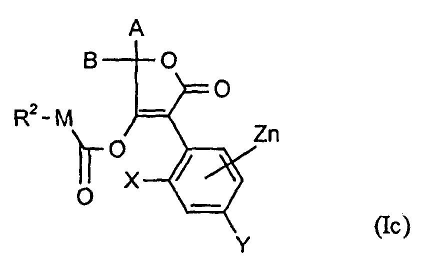 in welcher
A, B, X, Y, Z, R² und n die in Anspruch 1 angegebene Bedeutung haben,
und
M für Sauerstoff oder Schwefel steht,
Verbindungen der Formel (Ia) 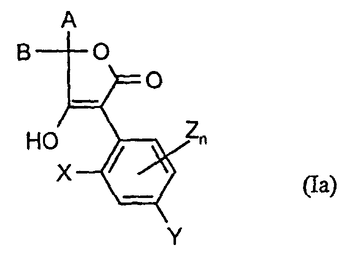 in welcher
A, B, X, Y, Z und n die oben angegebene Bedeutung haben, mit Chlorameisensäureester oder Chlorameisensäurethiolester der Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt.

5. Verbindungen der Formel (II) 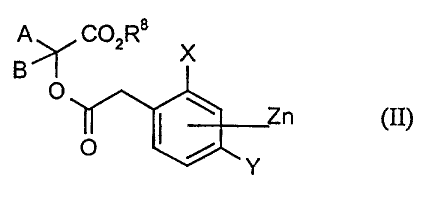 in welcher
A, B, X, Y, Z und n die in Anspruch 1 angegebene Bedeutung haben und
R⁸ für C₁-C₈-Alkyl steht.

6. Verfahren zur Herstellung von Verbindungen der Formel (II) gemäß Anspruch 6, **dadurch gekennzeichnet, dass** man
2-Hydroxycarbonsäure-Derivate der Formel (XIV), 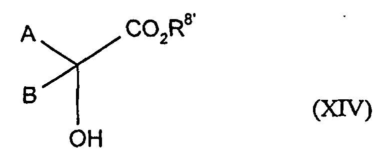 in welcher
R^{8'} für Wasserstoff oder Alkyl steht und
A und B die in Anspruch 1 angegebene Bedeutung haben,
mit Phenylessigsäurehalogeniden der Formel (XV) 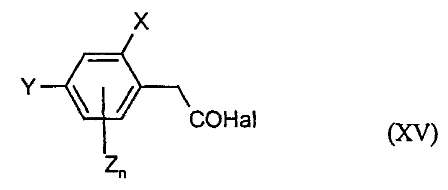 in welcher
X, Y, Z und n die in Anspruch 1 angegebene Bedeutung haben und
Hal für Chlor oder Brom steht,
acyliert und gegebenenfalls die dabei für R^{8'} = Wasserstoff gebildeten Verbindungen der Formel (IIa) 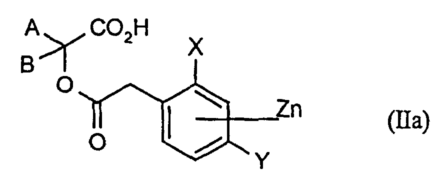 in welcher
A, B, X, Y, Z und n die oben angegebene Bedeutung haben,
verestert.

7. Schädlingsbekämpfungsmittel, **gekennzeichnet durch** einen Gehalt an mindestens einer Verbindung der Formel (I) gemäß Anspruch 1.

8. Verwendung von Verbindungen der Formel (I) gemäß Anspruch 1 zur Bekämpfung von Schädlingen.

9. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

10. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

## Claims

1. Compounds of the formula (I) 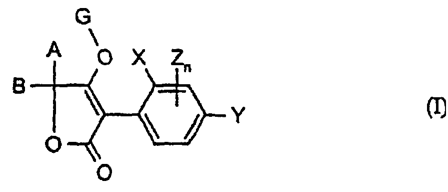 in which
A and B represent C₄-C₆-alkanediyl which is optionally monosubstituted or polysubstituted by identical or different substituents from the series consisting of halogen, C₁-C₈-alkyl, C₃-C₈-cycloalkyl, C₁-C₈-halogenoalkyl, C₁-C₆-alkoxy, C₁-C₆-alkylthio or phenyl and in which one or two carbon atoms which are not directly adjacent are replaced by the group 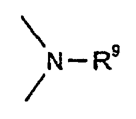 and/or oxygen and/or sulphur,
X represents C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
G represents hydrogen (a) or one of the groups 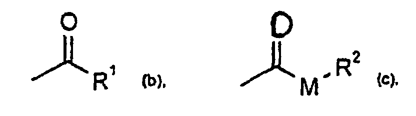 in which
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₂-C₈-alkyl, each of which is optionally monosubstituted or polysubstituted by halogen, or represents C₃-C₈-cycloalkyl which is optionally substituted by halogen, C₁-C₆-alkyl or C₁-C₆-alkoxy and in which one or two methylene groups which are not directly adjacent are optionally replaced by oxygen and/or sulphur,
or represents phenyl which is optionally monosubstituted to pentasubstituted by halogen, nitro, C₁-C₆-alkyl, C₁-C₆-alkoxy, C₁C₆-halogenoalkyl, C₁-C₆-halogenoalkoxy, C₁-C₆-alkylthio or C₁-C₆-alkylsulphonyl,
or represents phenyl-C₁-C₆-alkyl which is optionally monosubstituted to pentasubstituted by halogen, C₁-C₆-alkyl, C₁-C₆-alkoxy, C₁-C₆-halogenoalkyl or C₁-C₆-halogenoalkoxy,
or represents pyridyl, thienyl, furanyl, pyrazolyl, pyrimidyl or thiazolyl, each of which is optionally monosubstituted or disubstituted by halogen or C₁-C₆-alkyl,
or represents phenoxy-C₁-C₆-alkyl which is optionally monosubstituted to trisubstituted by halogen or C₁-C₆-alkyl,
or represents pyridinyloxy-C₁-C₆-alkyl, pyrimidinyloxy-C₁-C₆-alkyl or thiazolyloxy-C₁C₆-alkyl, each of which is optionally monosubstituted or disubstituted by halogen, amino or C₁-C₆-alkyl,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, each of which is optionally monosubstituted or polysubstituted by halogen, or represents C₃-C₈-cycloalkyl which is optionally monosubstituted or polysubstituted by halogen, C₁-C₄-alkyl or C₁-C₄-alkoxy,
or represents phenyl or benzyl, each of which is optionally monosubstituted to trisubstituted by halogen, nitro, C₁-C₆-alkyl, C₁-C₆-alkoxy or C₁-C₆-halogenoalkyl,
R⁹ represents hydrogen, Q, COQ or CO₂Q,
where
Q can assume the meanings which have been mentioned above for R² and
n represents 0, 1 or 2.

2. Compounds of the formula (I) according to Claim 1 in which
A and B represent C₄-C₅-alkanediyl which is optionally monosubstituted to tetrasubstituted by identical or differe substituents from the series consisting fluorine, chlorine, C₁-C₆-alkyl, C₃-C₆-cycloalkyl, C₁-C₆-halogenoalkyl, C₁-C₄-alkox C₁-C₄-alkylthio or phenyl and in which one two carbon atoms which are not direct adjacent are replaced by the group 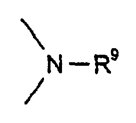 or by oxygen or by sulphur,
X represents C₁-C₄-alkyl, fluorine, chlorine, bromine or C₁-C₄-alkoxy,
Y represents hydrogen, C₁-C₄-alkyl, fluorine, chlorine, bromine, C₁-C₄-alkoxy or C₁-C₂-halogenoalkyl,
Z represents C₁-C₄-alkyl, fluorine, chlorine, bromine or C₁-C₄-alkoxy,
G represents hydrogen (a) or one of the groups 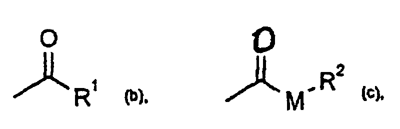 in which
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-alkyl, each of which is optionally monosubstituted to hexasubstituted by fluorine or chlorine,
or represents C₃-C₇-cycloalkyl which is optionally substituted by fluorine, chlorine, C₁-C₅-alkyl or C₁-C₅-alkoxy and in which one or two methylene groups which are not directly adjacent are optionally replaced by oxygen and/or sulphur,
or represents phenyl which is optionally monosubstituted to trisubstituted by fluorine, chlorine, bromine, nitro, C₁-C₄-alkyl, C₁-C₄-alkoxy, C₁-C₃-halogenoalkyl, C₁-C₃-halogenoalkoxy, C₁-C₄-alkylthio or C₁-C₄-alkylsulphonyl,
or represents phenyl-C₁-C₄-alkyl which is optionally monosubstituted to trisubstituted by fluorine, chlorine, bromine, C₁-C₄-alkyl, C₁-C₄-alkoxy, C₁-C₃-halogenoalkyl or C₁-C₃-halogenoalkoxy,
or represents pyridyl, thienyl, furanyl, pyrazolyl, pyrimidyl or thiazolyl, each of which is optionally monosubstituted or disubstituted by fluorine, chlorine, bromine or C₁-C₄-alkyl,
or represents phenoxy-C₁-C₅-alkyl which is optionally monosubstituted or disubstituted by fluorine, chlorine, bromine or C₁-C₄-alkyl,
or represents pyrimidinyloxy-C₁-C₅-alkyl, pyridinyloxy-C₁-C₅-alkyl or thiazolyloxy-C₁-C₅-alkyl, each of which is optionally monosubstituted or disubstituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally monosubstituted to hexasubstituted by fluorine or chlorine,
or represents C₃-C₇-cycloalkyl which is optionally monosubstituted to hexasubstituted by fluorine, chlorine, C₁-C₃-alkyl or C₁-C₃-alkoxy,
or represents phenyl or benzyl, each of which is optionally monosubstituted or disubstituted by fluorine, chlorine, bromine, nitro, C₁-C₄-alkyl, C₁-C₃-alkoxy or C₁-C₃-halogenoalkyl,
R⁹ represents hydrogen, Q, COQ or CO₂Q,
where
Q represents C₁-C₆-alkyl, phenyl or benzyl and
n represents 0 or 1.

3. Compounds of the formula (I) according to Claim 1, in which
A and B represent C₄-C₅-alkanediyl which is optionally monosubstituted to trisubstituted by identical or different substituents from the series consisting of fluorine, chlorine, methyl, ethyl, n-propyl, i-propyl, n-butyl, i-butyl, sec-butyl, tert-butyl, cyclohexyl, trifluoromethyl, methoxy, methylthio or phenyl and in which one carbon atom is replaced by the group 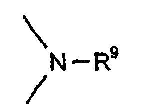 or by oxygen or by sulphur,
X represents methyl, ethyl, n-propyl, iso-propyl, fluorine, chlorine, bromine, methoxy or ethoxy,
Y represents hydrogen, methyl, ethyl, n-propyl, i-propyl, n-butyl, i-butyl, tert-butyl, fluorine, chlorine, bromine, methoxy, ethoxy or trifluoromethyl,
Z represents methyl, ethyl, propyl, i-propyl, n-butyl, i-butyl, tert-butyl, fluorine, chlorine, bromine, methoxy or ethoxy,
G represents hydrogen (a) or one of the groups 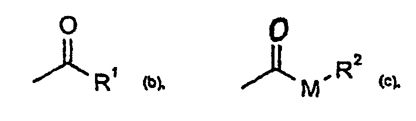 in which
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or poly-C₁-C₄-alkoxy-C₁-C₄-alkyl, each of which is optionally monosubstituted to trisubstituted by fluorine or chlorine,
or represents C₃-C₆-cycloalkyl which is optionally substituted by fluorine, chlorine, methyl, ethyl, propyl, i-propyl, butyl, i-butyl, t-butyl, methoxy, ethoxy, propoxy, i-propoxy, butoxy, i-butoxy, s-butoxy or t-butoxy and in which one methylene group is optionally replaced by oxygen or sulphur,
or represents phenyl which is optionally monosubstituted or disubstituted by fluorine, chlorine, bromine, methyl, ethyl, propyl, i-propyl, methoxy, ethoxy, trifluoromethyl, trifluoromethoxy, methylthio, ethylthio, methylsulphonyl, ethylsulphonyl or nitro,
or represents phenyl-C₁-C₃-alkyl which is optionally monosubstituted or disubstituted by fluorine, chlorine, bromine, methyl, ethyl, propyl, i-propyl, methoxy, ethoxy, trifluoromethyl or trifluoromethoxy,
or represents thienyl, furanyl or pyridyl, each of which is optionally monosubstituted or disubstituted by fluorine, chlorine, bromine, methyl or ethyl,
or represents phenoxy-C₁-C₄-alkyl which is optionally monosubstituted or disubstituted by fluorine, chlorine, methyl or ethyl,
or represents pyridyloxy-C₁-C₄-alkyl, pyrimidyloxy-C₁-C₄-alkyl or thiazolyloxy-C₁-C₄-alkyl, each of which is optionally monosubstituted or disubstituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, each of which is optionally monosubstituted to trisubstituted by fluorine or chlorine,
or represents C₃-C₆-cycloalkyl which is optionally monosubstituted to trisubstituted by fluorine, chlorine, methyl, ethyl, propyl, iso-propyl or methoxy,
or represents phenyl or benzyl, each of which is optionally monosubstituted or disubstituted by fluorine, chlorine, nitro, methyl, ethyl, propyl, i-propyl, methoxy, ethoxy or trifluoromethyl,
R⁹ represents hydrogen, Q, COQ or CO₂Q,
where
Q represents C₁-C₄-alkyl, phenyl or benzyl and
n represents 0 or 1.

4. Process for the preparation of compounds of the formula (I) according to Claim 1, **characterized in that**,
(A) to obtain compounds of the formula (Ia) 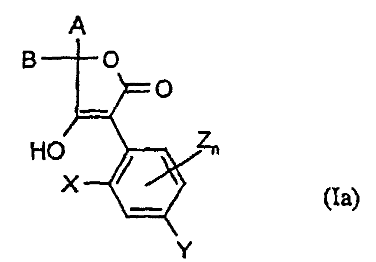 in which
A, B, X, Y, Z and n have the meanings given in Claim 1,
carboxylic esters of the formula (II) 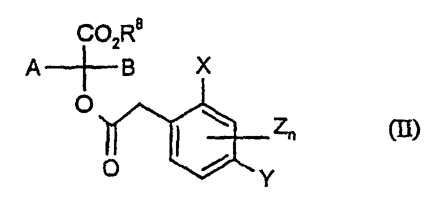 in which
A, B, X, Y, Z and n have the abovementioned meanings and
R⁸ represents alkyl
are subjected to an intramolecular condensation reaction in the presence of a diluent and in the presence of a base;
or,
(B) to obtain compounds of the formula (Ib) 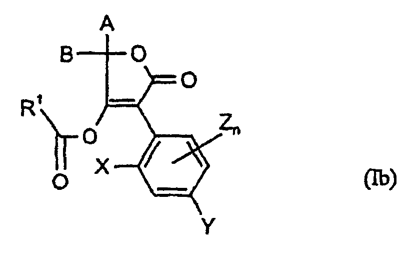 in which
A, B, X, Y, Z, R¹ and n have the meanings given in Claim 1,
compounds of the formula (Ia) 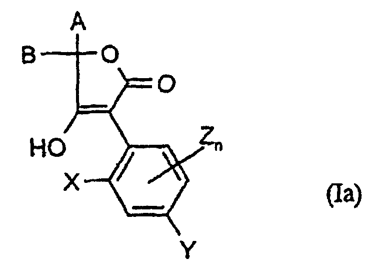 in which
A, B, X, Y, Z and n have the abovementioned meanings,
are reacted
α) with acid halides of the formula (III) 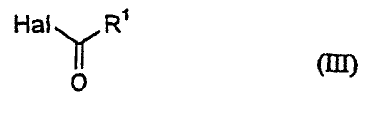 in which
R¹ has the abovementioned meanings and
Hal represents halogen,
if appropriate in the presence of a diluent and if appropriate in the presence of an acid-binding agent,
or
β) with carboxylic anhydrides of the formula (IV)
R¹-CO-O-CO-R¹ (IV)
in which
R¹ has the abovementioned meanings,
if appropriate in the presence of a diluent and if appropriate in the presence of an acid-binding agent;
or
(C) to obtain compounds of the formula (Ic) 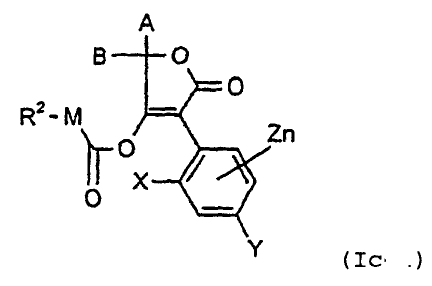 in which
A, B, X, Y, Z, R² and n have the meanings given in Claim 1,
and
Mrepresents oxygen or sulphur,
compounds of the formula (Ia) 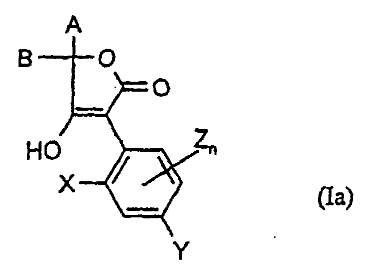 in which
A, B, X, Y, Z and n have the abovementioned meanings,
are reacted with chloroformic esters or chloroformic thioesters of the formula (V)
R²-M-CO-Cl (V)
in which
R² and M have the abovementioned meanings,
if appropriate in the presence of a diluent and if appropriate in the presence of an acid-binding agent.

5. Compounds of the formula (II) 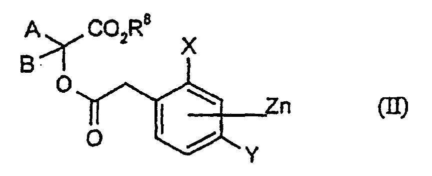 in which
A, B, X, Y, Z and n have the meanings given in Claim 1 and
R⁸ represents C₁-C₈-alkyl.

6. Process for the preparation of compounds of the formula (II) according to Claim 5, **characterized in that**
2-hydroxycarboxylic acid derivatives of the formula (XIV) 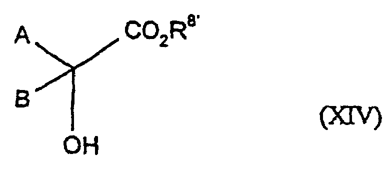 in which
R⁸' represents hydrogen or alkyl and
A and B have the meanings given in Claim 1,
are acylated with phenylacetyl halides of the formula (XV) 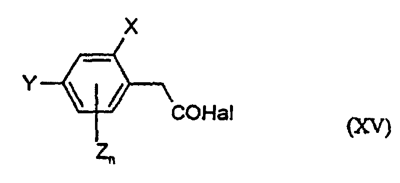 in which
X, Y, Z and n have the meanings given in Claim 1 and
Halrepresents chlorine or bromine,
and, in the event that R^{8'} = hydrogen, the compounds formed, of the formula (IIa) 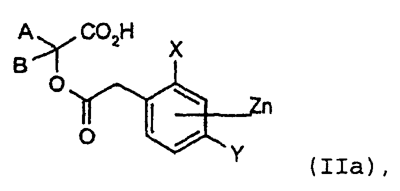 in which
A, B, X, Y, Z and n have the abovementioned meanings,
are, if appropriate, esterified.

7. Pesticides, **characterized in that** they comprise at least one compound of the formula (I) according to Claim 1.

8. Use of compounds of the formula (I) according to Claim 1 for combating pests.

9. Method of combating pests, **characterized in that** compounds of the formula (I) according to Claim 1 are allowed to act on pests and/or their environment.

10. Process for the preparation of pesticides, **characterized in that** compounds of the formula (I) according to Claim 1 are mixed with extenders and/or surface-active agents.

## Revendications

1. Composés de formule (I) 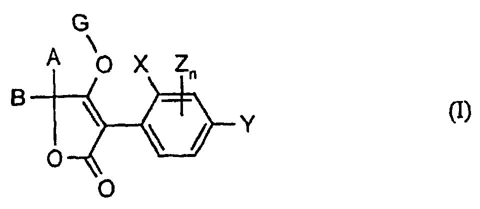 dans laquelle
A et B représentent un groupe alcanediyle portant éventuellement un ou plusieurs substituants, identiques ou différents, halogéno, alkyle en C₁ à C₈, cycloalkyle en C₃ à C₈, halogénalkyle en C₁ à C₈, alkoxy en C₁ à C₆, alkylthio en C₁ à C₆ ou phényle, et dans lequel un ou deux atomes de carbone non contigus sont remplacés par le groupe 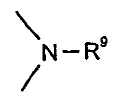 et/ou par de l'oxygène et/ou par du soufre,
X est un groupe alkyle en C₁ à C₆, un halogène ou un groupe alkoxy en C₁ à C₆,
Y représente l'hydrogène, un groupe alkyle en C₁ à C₆, un halogène, un groupe alkoxy en C₁ à C₆ ou halogén-alkyle en C₁ à C₃,
Z est un groupe alkyle en C₁ à C₆, un halogène ou un groupe alkoxy en C₁ à C₆,
G représente l'hydrogène (a) ou l'un des groupes 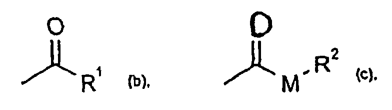 dans lesquels
M est l'oxygène ou le soufre,
R¹ représente un groupe alkyle en C₁ à C₂₀, alcényle en C₂ à C₂₀, (alkoxy en C₁ à C₈)-(alkyle en C₁ à C₈), (alkylthio en C₁ à C₈)-(alkyle en C₁ à C₈), poly(alkoxy en C₁ à C₈)-(alkyle en C₂ à C₈) portant chacun, le cas échéant, un ou plusieurs substituants halogéno, ou un groupe cycloalkyle en C₃ à C₈ portant éventuellement un substituant halogéno, alkyle en C₁ à C₆ ou alkoxy en C₁ à C₆ et dans lequel un ou deux groupes méthylène non contigus sont éventuellement remplacés par de l'oxygène et/ou par du soufre,
un groupe phényle portant éventuellement un à cinq substituants halogéno, nitro, alkyle en C₁ à C₆, alkoxy en C₁ à C₆, halogénalkyle en C₁ à C₆, halogén-alkoxy en C₁ à C₆, alkylthio en C₁ à C₆ ou alkylsulfonyle en C₁ à C₆,
un groupe phényl-(alkyle en C₁ à C₆) portant éventuellement un à cinq substituants halogéno, alkyle en C₁ à C₆, alkoxy en C₁ à C₆, halogénalkyle en C₁ à C₆ ou halogénalkoxy en C₁ à C₆,
un groupe pyridyle, thiényle, furannyle, pyrazolyle, pyrimidyle ou thiazolyle portant chacun, le cas échéant, un ou deux substituants halogéno ou alkyle en C₁ à C₆,
un groupe phénoxy-(alkyle en C₁ à C₆) portant éventuellement un à trois substituants halogéno ou alkyle en C₁ à C₆, ou bien
un groupe pyridinyloxy-(alkyle en C₁ à C₆), pyrimidinyloxy-(alkyle en C₁ à C₆) ou thiazolyloxy-(alkyle en C₁ à C₆) portant chacun, le cas échéant, un ou deux substituants halogéno, amino ou alkyle en C₁ à C₆,
R² représente un groupe alkyle en C₁ à C₂₀, alcényle en C₂ à C₂₀, (alkoxy en C₁ à C₈)-(alkyle en C₂ à C₈) ou poly-(alkoxy en C₁ à C₈)-(alkyle en C₂ à C₈) portant chacun, le cas échéant, un ou plusieurs substituants halogéno, un groupe cycloalkyle en C₃ à C₈ portant éventuellement un ou plusieurs substituants halogéno, alkyle en C₁ à C₄ ou alkoxy en C₁ à C₄, ou bien
un groupe phényle ou un groupe benzyle portant chacun, le cas échéant, un à trois substituants halogéno, nitro, alkyle en C₁ à C₆, alkoxy en C₁ à C₆ ou halogén-alkyle en C₁ à C₆,
R⁹ représente l'hydrogène, un groupe Q, COQ ou CO₂Q, où
Q peut avoir les définitions mentionnées ci-dessus pour R² et
n a la valeur 0, 1 ou 2.

2. Composés de formule (I) suivant la revendication 1, formule dans laquelle
A et B représentent un groupe alcanediyle en C₄ ou C₅ portant éventuellement un à quatre substituants, identiques ou différents, fluoro, chloro, alkyle en C₁ à C₆, cycloalkyle en C₃ à C₆, halogénalkyle en C₁ à C₆, alkoxy en C₁ à C₄, alkylthio en C₁ à C₄ ou phényle, dont un ou deux atomes de carbone non contigus sont remplacés par le groupe 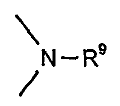 ou par de l'oxygène ou du soufre,
X est un groupe alkyle en C₁ à C₄, le fluor, le chlore, le brome ou un groupe alkoxy en C₁ à C₄,
Y représente l'hydrogène, un groupe alkyle en C₁ à C₄, le fluor, le chlore, le brome, un groupe alkoxy en C₁ à C₄ ou halogénalkyle en C₁ ou C₂,
Z est un groupe alkyle en C₁ à C₄, le fluor, le chlore, le brome ou un groupe alkoxy en C₁ à C₄,
G représente l'hydrogène (a) ou l'un des groupes 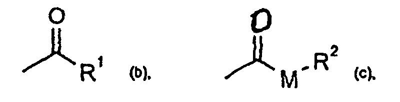 dans lesquels
M représente l'oxygène ou le soufre,
R¹ est un groupe alkyle en C₁ à C₁₆, alcényle en C₂ à C₁₆, (alkoxy en C₁ à C₆)-(alkyle en C₁ à C₆), (alkylthio en C₁ à C₆)-(alkyle en C₁ à C₆), poly(alkoxy en C₁ à C₆)-(alkyle en C₁ à C₆) portant chacun, le cas échéant, un à six substituants fluoro ou chloro, ou un groupe cycloalkyle en C₃ à C₇ éventuellement substitué par du fluor, du chlore, un radical alkyle en C₁ à C₅ ou alkoxy en C₁ à C₅, dans lequel, le cas échéant, un ou deux groupes méthylène non contigus sont remplacés par de l'oxygène et/ou par du soufre,
un groupe phényle portant éventuellement un à trois substituants fluoro, chloro, bromo, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un groupe phényl-(alkyle en C₁ à C₄) portant éventuellement un à trois substituants fluoro, chloro, bromo, alkyle en C₁ à C₄, alkoxy en C₁ à C₄, halogénalkyle en C₁ à C₃ ou halogénalkoxy en C₁ à C₃,
un groupe pyridyle, thiényle, furannyle, pyrazolyle, pyrimidyle ou thiazolyle portant chacun, le cas échéant, un ou deux substituants fluoro, chloro, bromo ou alkyle en C₁ à C₄,
un groupe phénoxy-(alkyle en C₁ à C₅) portant éventuellement un ou deux substituants fluoro, chloro, bromo ou alkyle en C₁ à C₄, ou bien
un groupe pyrimidinyloxy (alkyle en C₁ à C₅), pyridinyloxy-(alkyle en C₁ à C₅) ou thiazolyloxy-(alkyle en C₁ à C₅) portant chacun, le cas échéant, un ou deux substituants fluoro, chloro, bromo, amino ou alkyle en C₁ à C₄,
R² représente un groupe alkyle en C₁ à C₁₆, alcényle en C₂ à C₁₆, (alkoxy en C₁ à C₆)-(alkyle en C₂ à C₆) ou poly-(alkoxy en C₁ à C₆)-(alkyle en C₂ à C₆) portant chacun, le cas échéant, un à six substituants fluoro ou chloro,
un groupe cycloalkyle en C₃ à C₇ portant éventuellement un à six substituants fluoro, chloro, alkyle en C₁ à C₃ ou alkoxy en C₁ à C₃, ou bien
un groupe phényle ou un groupe benzyle portant chacun, le cas échéant, un ou deux substituants fluoro, chloro, bromo, nitro, alkyle en C₁ à C₄, alkoxy en C₁ à C₃ ou halogénalkyle en C₁ à C₃,
R⁹ représente l'hydrogène, un groupe Q, COQ ou CO₂Q, où
Q représente un groupe alkyle en C₁ à C₆, phényle ou benzyle et
n a la valeur 0, 1 ou 2.

3. Composés de formule (I) suivant la revendication 1, formule dans laquelle
A et B représentent un groupe alcanediyle en C₄ ou C₅ portant éventuellement un à trois substituants, identiques ou différents, fluoro, chloro, méthyle, éthyle, n-propyle, isopropyle, n-butyle, isobutyle, sec.-butyle, tertio-butyle, cyclohexyle, trifluorométhyle, méthoxy, méthylthio ou phényle, dans lequel un atome de carbone est remplacé par le groupe 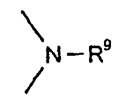 ou par de l'oxygène ou du soufre,
X est un groupe méthyle, éthyle, n-propyle, isopropyle, le fluor, le chlore, le brome, un groupe méthoxy ou éthoxy,
Y représente l'hydrogène, un groupe méthyle, éthyle, n-propyle, isopropyle, n-butyle, isobutyle, tertio-butyle, le fluor, le chlore, le brome, un groupe méthoxy, éthoxy ou trifluorométhyle,
Z est un groupe méthyle, éthyle, propyle, isopropyle, n-butyle, isobutyle, tertio-butyle, le fluor, le chlore, le brome, un groupe méthoxy ou éthoxy,
G représente l'hydrogène (a) ou l'un des groupes 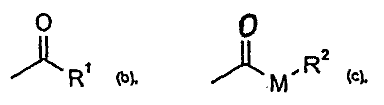 dans lesquels
M est l'oxygène ou le soufre,
R¹ représente un groupe alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₁ à C₄) portant chacun, le cas échéant, un à trois substituants fluoro ou chloro,
ou un groupe cycloalkyle en C₃ à C₆ éventuellement substitué par du fluor, du chlore, un radical méthyle, éthyle, propyle, isopropyle, butyle, isobutyle, tertio-butyle, méthoxy, éthoxy, propoxy, isopropoxy, butoxy, isobutoxy, sec.-butoxy ou tertio-butoxy, dans lequel, le cas échéant, un groupe méthylène est remplacé par de l'oxygène ou du soufre,
un groupe phényle portant éventuellement un ou deux substituants fluoro, chloro, bromo, méthyle, éthyle, propyle, isopropyle, méthoxy, éthoxy, trifluorométhyle, trifluorométhoxy, méthylthio, éthylthio, méthylsulfonyle, éthylsulfonyle ou nitro,
un groupe phényl-(alkyle en C₁ à C₃) portant éventuellement un ou deux substituants fluoro, chloro, bromo, méthyle, éthyle, propyle, isopropyle, méthoxy, éthoxy, trifluorométhyle ou trifluorométhoxy,
un groupe thiényle, furannyle ou pyridyle portant chacun, le cas échéant, un ou deux substituants fluoro, chloro, bromo, méthyle ou éthyle,
un groupe phénoxy-(alkyle en C₁ à C₄) portant éventuellement un ou deux substituants fluoro, chloro, méthyle ou éthyle, ou
un groupe pyridyloxy-(alkyle en C₁ à C₄), pyrimidyloxy-(alkyle en C₁ à C₄) ou thiazolyloxy-(alkyle en C₁ à C₄) portant chacun, le cas échéant, un ou deux substituants fluoro, chloro, amino, méthyle ou éthyle,
R² représente un groupe alkyle en C₁ à C₁₄, alcényle en C₂ à C₁₄, (alkoxy en C₁ à C₄)-(alkyle en C₂ à C₆) ou poly-(alkoxy en C₁ à C₄)-(alkyle en C₂ à C₆) portant chacun éventuellement un à trois substituants fluoro ou chloro,
un groupe cycloalkyle en C₃ à C₆ portant éventuellement un à trois substituants fluoro, chloro, méthyle, éthyle, propyle, isopropyle ou méthoxy,
un groupe phényle ou un groupe benzyle portant chacun, le cas échéant, un ou deux substituants fluoro, chloro, nitro, méthyle, éthyle, propyle, isopropyle, méthoxy, éthoxy ou trifluorométhyle,
R⁹ représente l'hydrogène, un groupe Q, COQ ou CO₂Q, où
Q représente un radical alkyle en C₁ à C₄, phényle ou benzyle et
n a la valeur 0 ou 1.

4. Procédé de production de composés de formule (I) suivant la revendication 1, **caractérisé en ce que** :
(A) pour obtenir des composés de formule (Ia) 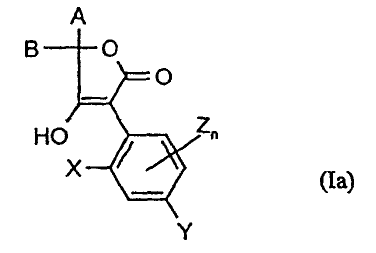 dans laquelle
A, B, X, Y, Z et n ont la définition indiquée dans la revendication 1,
on effectue la condensation intramoléculaire d'esters d'acides carboxyliques de formule (II) 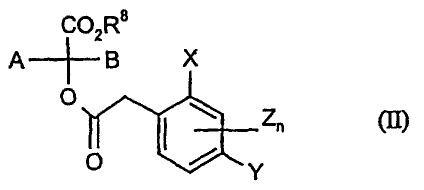 dans laquelle
A, B, X, Y, Z et n ont la définition indiquée ci-dessus, et
R⁸ est un groupe alkyle,
en présence d'un diluant et en présence d'une bas ou bien
(B) pour obtenir des composés de formule (Ib) 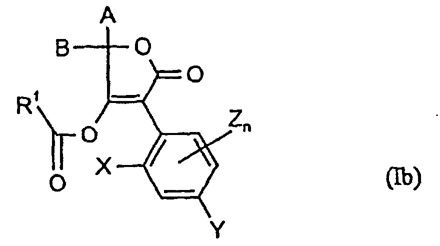 dans laquelle
A, B, X, Y, Z, R¹ et n ont la définition indiquée dans la revendication 1,
on fait réagir des composés de formule (Ia) 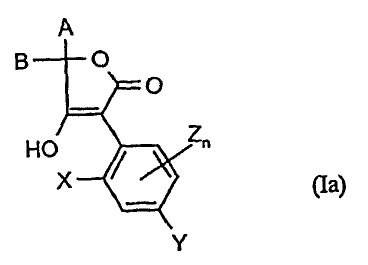 dans laquelle
A, B, X, Y, Z et n ont la définition indiquée ci-dessus,
α) avec des halogénures d'acides de formule (III) 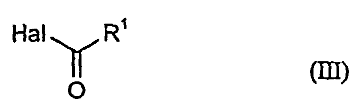 dans laquelle
R¹ a la définition indiquée ci-dessus et
Hal représente un halogène,
éventuellement en présence d'un diluant et, le cas échéant en présence d'un accepteur d'acide, ou bien
β) avec des anhydrides d'acides carboxyliques de formule (IV)
R¹-CO-O-CO-R¹ (IV)
dans laquelle
R¹ a la définition indiquée ci-dessus,
éventuellement en présence d'un diluant et, le cas échéant en présence d'un accepteur d'acide ;
ou bien
(C) pour obtenir des composés de formule (Ic) 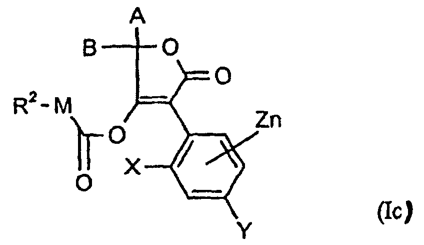
dans laquelle
A, B, X, Y, Z, R² et n ont la définition indiquée dans la revendication 1,
et
M représente l'oxygène ou le soufre,
on fait réagir des composés de formule (Ia) 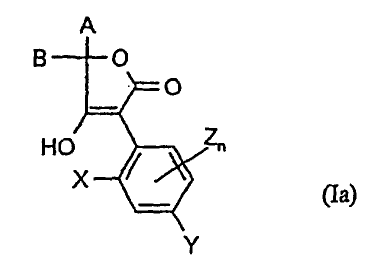 dans laquelle
A, B, X, Y, Z et n ont la définition indiquée ci-dessus,
avec un ester d'acide chloroformique ou un thio-ester d'acide chloroformique de formule (V)
R²-M-CO-Cl (V)
dans laquelle
R² et M ont la définition indiquée ci-dessus,
éventuellement en présence d'un diluant et, le cas échéant en présence d'un accepteur d'acide.

5. Composés de formule (II) 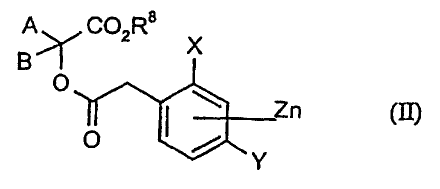 dans laquelle
A, B, X, Y, Z et n ont la définition indiquée dans la revendication 1 et
R⁸ est un groupe alkyle en C₁ à C₈.

6. Procédé de production de composés de formule (II) suivant la revendication 6, **caractérisé en ce qu'**on acyle des dérivés d'acides 2-hydroxycarboxyliques de formule (XIV) 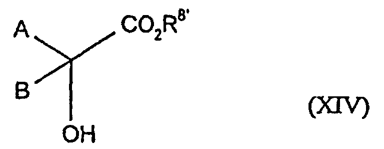 dans laquelle
R⁸ représente l'hydrogène ou un groupe alkyle et
A et B ont la définition indiquée dans la revendication 1,
avec des halogénures d'acides phénylacétiques de formule (XV) 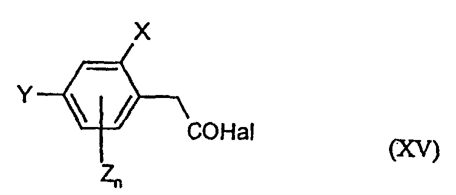 dans laquelle
X, Y, Z et n ont la définition indiquée dans la revendication 1 et
Hal représente le chlore ou le brome,
et, le cas échéant, on estérifie les composés ainsi formés lorsque R⁸' est l'hydrogène, de formule (IIa) 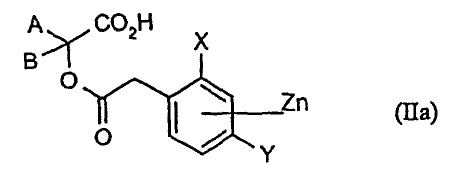 dans laquelle
A, B, X, Y, Z et n ont la définition indiquée ci-dessus.

7. Compositions pesticides, **caractérisées par** une teneur en au moins un composé de formule (I) suivant la revendication 1.

8. Utilisation de composés de formule (I) suivant la revendication 1 pour combattre des parasites.

9. Procédé pour combattre des parasites, **caractérisé en ce qu'**on fait agir des composés de formule (I) suivant la revendication 1 sur les parasites et/ou sur leur milieu.

10. Procédé de préparation de compositions pesticides, **caractérisé en ce qu'**on mélange des composés de formule (I) suivant la revendication 1 avec des diluants et/ou des agents tensio-actifs.
